(19) Europäisches Patentamt / European Patent Office / Office européen des brevets

(11) **EP 2 716 223 A1**

(12) **EUROPEAN PATENT APPLICATION**
published in accordance with Art. 153(4) EPC

(43) Date of publication:
**09.04.2014 Bulletin 2014/15**

(51) Int Cl.:
*A61B 6/00* (2006.01)   *A61B 6/10* (2006.01)

(21) Application number: **12792595.6**

(22) Date of filing: **30.05.2012**

(86) International application number:
**PCT/JP2012/003559**

(87) International publication number:
**WO 2012/164932 (06.12.2012 Gazette 2012/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2011   JP 2011120434**
**30.05.2011   JP 2011120435**
**30.05.2011   JP 2011120437**
**30.05.2011   JP 2011120438**

(71) Applicant: **FUJIFILM Corporation**
**Tokyo 106-8620 (JP)**

(72) Inventors:
• **NAKATSUGAWA, Haruyasu**
**Ashigarakami-gun**
**Kanagawa 258-8538 (JP)**
• **NISHINO, Naoyuki**
**Ashigarakami-gun**
**Kanagawa 258-8538 (JP)**
• **YOSHIDA, Yutaka**
**Ashigarakami-gun**
**Kanagawa 258-8538 (JP)**
• **OHTA, Yasunori**
**Ashigarakami-gun**
**Kanagawa 258-8538 (JP)**
• **KAMIYA, Takeshi**
**Ashigarakami-gun**
**Kanagawa 258-8538 (JP)**

(74) Representative: **Klunker . Schmitt-Nilson . Hirsch**
**Patentanwälte**
**Destouchesstrasse 68**
**80796 München (DE)**

(54) **DEVICE AND METHOD FOR ACQUIRING RADIATION EXPOSURE LEVEL, AND PROGRAM**

(57)      [Problems to be Solved]
In a case where a radiological imaging for a subject is performed continuously, obtaining a radiation exposure dose for each region of the subject.

[Means for Solving the Problems]
In a case where a radiation exposure dose of a subject is obtained in a radiation exposure dose obtaining apparatus (40) based on a radiation image signal of each frame detected by a radiation image detector (12) by continuously performing radiological imaging of the subject due to the continuous radiological imaging, an area corresponding to a region of the subject is set in an image represented by the radiation image signal and a radiation exposure dose is obtained for each set area.

**FIG.1**

EP 2 716 223 A1

**Description**

Technical Field

The present invention relates to a radiation exposure dose obtaining apparatus, method, and program that obtains a radiation exposure dose of a subject to be injected with a contrast agent based on a radiological moving picture signal which is a collection of radiation image signals of each frame obtained through continuous radiological imaging of the subject.

Background Art

Recently, it has been regarded as important to manage exposure doses of a patient due to radiological imaging in the medical front where radiological imaging is performed. As the relationship between the exposure dose of the patient due to radiological imaging and a disease such as cancer is drawing attention, it is necessary to maintain the exposure dose of a patient due to radiological imaging and to understand and manage the radiation exposure dose cumulatively received by the patient.

As for the method of measuring the radiation exposure dose received by a patient during radiological imaging, for example, a method in which a radiation source that emits radiation is provided with an area dosimeter and the radiation exposure dose received by the patient is obtained based on the value measured by the dosimeter is proposed

The employment of such method, however, requires a new dosimeter for measuring the exposure dose of the patient, causing a problem of cost increase.

Consequently, instead of providing such dosimeter described above, it is proposed that the exposure dose of a patient is obtained based on an image signal detected by a radiation image detector that detects a radiation image of the patient (refer to, for example, Patent Document 1).

[Prior Art Documents]

[Patent Documents]

Patent Document 1: Japanese Patent No. 4387644

Disclosure of the Invention

Patent Document 1 proposes that an exposure dose of a patient is obtained in the radiological imaging of a general still image. In the actual medical front, however, radiological imaging of moving pictures, such as fluoroscopic images, may be performed, as well as such radiological imaging of still images.

Further, the same radiation exposure dose may give different amounts of impact to each region of a human body and, for example, it is said that the impact of the same amount of radiation exposure on the lung or stomach is two times as great as that on the liver or esophagus. Therefore, when performing risk management of diseases, such as cancer, more accurate management may be made by figuring out the exposure dose of each region, in addition to the exposure dose of the entire human body.

Patent Document 1 proposes neither the acquisition of the exposure dose throughout the radiological imaging of a fluoroscopic image nor the acquisition of the radiation exposure dose for each region of the subject.

In the radiological imaging of such fluoroscopic images, there may be a case where a contrast agent is injected, for example, into a blood vessel to obtain and observe a contrast agent image. In such a case, if the exposure dose of the patient is tried to be obtained based simply on the image signal of the radiation image detector, there arises a concern that the calculated value may possibly differ from the actual exposure dose of the patient. That is, the radiation is absorbed by the contrast agent and the amount of radiation reached the radiation image detector is reduced.

Further, in the case where a contrast agent is injected during the radiological imaging of a fluoroscopic image, the contrast agent flows through a blood vessel or the like without remaining in one place, so that not all of the frames of the radiological imaging of the fluoroscopic image but some of them will include an image of the contrast agent. Therefore, it is necessary to obtain the exposure dose of the patient throughout the radiological imaging of the fluoroscopic image by taking into account the point described above.

Patent Document 1 proposes neither the acquisition of the exposure dose throughout the radiological imaging of a fluoroscopic image nor the acquisition of the exposure dose considering the inclusion of an image of a contrast agent.

In view of the circumstances described above, it is a first object of the present invention to provide a radiation exposure dose obtaining apparatus, method, and program capable of obtaining a radiation exposure dose for each region of a subject when radiological imaging of the subject is performed continuously.

It is a second, third, and fourth object of the present invention to provide a radiation exposure dose obtaining apparatus, method, and program capable of obtaining a radiation exposure dose of a subject more accurately even in a case where radiological imaging of a subject to be injected with a contrast agent is performed in series.

A first radiation exposure dose obtaining apparatus of the present invention includes a radiation exposure dose obtaining unit that obtains, based on a radiation image signal of each frame detected by a radiation image detector through continuous radiological imaging of a subject, a radiation exposure dose of the subject due to the continuous radiological imaging and an area setting unit that sets one or more areas corresponding to a region of the subject in an image represented by the radiation image signal, in which the radiation exposure dose obtaining unit obtains the radiation exposure dose for each area.

In the radiation exposure dose obtaining apparatus described above, the area setting unit may segment the image represented by the radiation image signal into a plurality of predetermined segmented areas and associate each segmented area with a region of the subject or may identify an area corresponding to a region of the subject in the image represented by the radiation image signal by image recognition processing and associate the identified area with the region of the subject.

The object of the first radiation exposure dose obtaining apparatus described above is to measure and manage an exposure dose for each region (organ) as the radiation absorption coefficient and the allowable cumulative dose differ from region (organ) to region (organ).

Because there is a concern that children are more influenced by radiation exposure than adults, it is important to measure and manage the exposure dose more accurately. Bodies of children grow remarkably and the size of an organ in an image is likely to be changed with time even if the radiological imaging is performed under the same condition. Further, even for radiological imaging of an adult, the size of an organ in images may possibly be changed due to difference in radiological imaging equipment or imaging condition. Therefore, an area is set in association with a region means that the area may be variable for each imaging.

In the radiation exposure dose obtaining apparatus described above, there may be a case, for example, where a contrast agent is injected into a blood vessel or the like to obtain and observe a contrast agent image of the blood vessel or the like. In such a case, if an exposure dose of the subject is obtained based simply on an image signal of the radiation image detector, an accurate exposure dose can not be obtained. That is, the radiation applied to the patient is absorbed by the contrast agent, as well as the body, before reaching the radiation image detector. Consequently, the exposure dose obtained based on the radiation image signal which reflects such absorption of the radiation by the contrast agent is not accurate.

Further, in the case where a contrast agent is injected for radiological imaging of a fluoroscopic image, the contrast agent flows through a blood vessel or the like without remaining in one place, so that not all of the frames of the radiological imaging of the fluoroscopic image but some of them will capture an image of the contrast agent. Therefore, it is preferable that the exposure dose of the patient over the throughout the imaging of the fluoroscopic image is obtained by taking into account the point described above.

Therefore, it is preferable that the radiation exposure dose obtaining apparatus described above includes a contrast agent frame identification unit that identifies a frame which includes an image signal representing an image of a contrast agent injected into the subject as a contrast agent frame from the radiation image signal of each frame and the radiation exposure dose obtaining unit performs a correction on a radiation exposure dose for the contrast agent frame to bring the radiation exposure dose close to an actual value.

Preferably, the radiation exposure dose obtaining unit obtains the radiation exposure dose based on an E.I. (Exposure Index) of the radiation image signal, but any value other than this may also be used as long as it serves as an index for determining how much of radiation is detected in each frame, such as an average value of signal values in each frame or a value calculated by considering the histogram. The "value calculated based on an E.I." as used herein may be the value of the E.I. itself or a value obtained by making a certain correction to the E.I.

A first radiation exposure dose obtaining method of the present invention obtains, based on a radiation image signal detected by a radiation image detector through continuous radiological imaging of a subject, a radiation exposure dose of the subject due to the continuous radiological imaging, comprising the steps of: setting one or more areas corresponding to a region of the subject in an image represented by the radiation image signal; and obtaining the radiation exposure dose for each area.

The radiation exposure dose obtaining method described above may be provided as a program for causing a computer to perform the method.

A second radiation exposure dose obtaining apparatus of the present invention includes a radiation exposure dose obtaining unit that obtains, based on a radiation moving picture signal, which is a collection of a radiation image signal of each frame detected by a radiation image detector through continuous radiological imaging of a subject, a radiation exposure dose of the subject due to the continuous radiological imaging, in which the radiation exposure dose obtaining unit calculates a signal evaluation value that serves as an index for determining how much of radiation is detected over the entire radiation moving picture signal by a formula (1) and obtains the radiation exposure dose of the subject based on the signal evaluation value of the radiation moving picture signal:

$$Rm = Rf \times N \quad ----------- \quad (1).$$

A second radiation exposure dose obtaining method of the present invention obtains, based on a radiation moving picture signal, which is a collection of a radiation image signal of each frame detected by a radiation image detector through continuous radiological imaging of a subject, a radiation exposure dose of the subject due to the continuous radiological imaging, the method including the steps of: calculating a signal evaluation value that serves as an index for determining how much of radiation is detected over the entire radiation moving picture signal by a formula (1); and obtaining the radiation exposure dose of the subject based on the signal evaluation value of the radiation moving picture signal:

$$Rm = Rf \times N \quad ----------- \quad (1).$$

In the radiation exposure dose obtaining apparatus and method described above, the following have the following meanings:

$Rm$: the signal evaluation value of the radiation moving picture signal;
$N$: the number of frames of the entire radiation moving picture signal; and
$Rf$: the signal evaluation value of the radiation image signal of a predetermined one frame in the radiation moving picture signal.

The formula (1) given above will now be described in detail. The signal evaluation value of the radiation moving picture signal is a value that serves as an index for determining how much of radiation is detected over the entire radiation moving picture signal, and the signal evaluation value is calculated based on a signal evaluation value of radiation image signals of predetermined one frame (a value that serves as an index for determining how much of radiation is detected in the predetermined one frame).

The "signal evaluation value of radiation image signals of predetermined one frame" is preferable to be a maximum signal evaluation value among those calculated for each radiation image signal or a signal evaluation value of radiation image signals of the first frame of the radiation moving picture signal, but any other form of may be employed as long as it is a signal evaluation value of radiation image signals for one frame deemed to be free of error factor, such as a signal evaluation value of radiation image signals of one particular frame which does not include any error factor such as the contrast agent described above, an average value of signal evaluation values of radiation image signals of a plurality of frames that do not include any error factor, or the like.

The signal evaluation value of radiation image signals of a predetermined one frame is preferable to be a value calculated based on an E.I. (Exposure Index), but any value other than this may also be used as long as it serves as an index for determining how much of radiation is detected in each frame, such as an average value of signal values in each frame or a value calculated by considering the histogram. The "value calculated based on an E.I." as used herein may be the value of the E.I. itself or a value obtained by making a certain correction to the E.I.

The radiation exposure dose obtaining method described above may be provided as a program for causing a computer to perform the method.

A third radiation exposure dose obtaining apparatus of the present invention includes: a radiation exposure dose obtaining unit that obtains, based on a radiation image signal of each frame detected by a radiation image detector through continuous radiological imaging of a subject to be injected with a contrast agent, a radiation exposure dose of the subject due to the continuous radiological imaging; and a contrast agent frame identification unit that identifies a frame which includes an image signal representing an image of the contrast agent as a contrast agent frame from the radiation image signal of each frame, in which the radiation exposure dose obtaining unit calculates a signal evaluation value of a radiation moving picture signal, which is a collection of the radiation image signal of each frame, by a formula (11) and obtains the radiation exposure dose of the subject due to the continuous radiological imaging based on the signal evaluation value of the radiation moving picture signal:

$$Rm = Rs \times N/(N-M) \quad ----------- \quad (11).$$

A third radiation exposure dose obtaining method of the present invention obtains, based on a radiation image signal of each frame detected by a radiation image detector through continuous radiological imaging of a subject to be injected with a contrast agent, a radiation exposure dose of the subject due to the continuous radiological imaging, the method comprising the steps of: identifying a frame which includes an image signal representing an image of the contrast agent as a contrast agent frame from the radiation image signal of each frame; calculating a signal evaluation value of a radiation moving picture signal, which is a collection of the radiation image signal of each frame, by a formula (11); and obtaining the radiation exposure dose of the subject due to the continuous radiological imaging based on the signal evaluation value of the radiation moving picture signal:

$$Rm = Rs \times N/(N-M) \quad ---------- \quad (11).$$

In the radiation exposure dose obtaining apparatus and method described above, the following have the following meanings:

Rm: the signal evaluation value of the radiation moving picture signal;
N: the number of frames of the entire radiation moving picture signal;
M: the number of contrast agent frames in the radiation moving picture signal;
Rs: the sum of signal evaluation value of radiation image signal of each frame other than the contrast agent frame.

The formula (11) given above will now be described in detail. The signal evaluation value of the radiation moving picture signal is a value that serves as an index for determining how much of radiation is detected over the entire radiation moving picture signal, and the signal evaluation value is calculated based on a signal evaluation value of radiation image signals of each frame (a value that serves as an index for determining how much of radiation is detected in each frame).

The signal evaluation value of radiation image signals of each frame is preferable to be a value calculated based on an E. I. (Exposure Index), but any value other than this may also be used as long as it serves as an index for determining how much of radiation is detected in each frame, such as an average value of signal values in each frame or a value calculated by considering the histogram. The "value calculated based on an E.I." as used herein may be the value of the E.I. itself or a value obtained by making a certain correction to the E.I.

Further, an average value Ra of signal evaluation values of radiation image signals of each frame other than a contrast agent frame in the radiation moving picture signal may be obtained as in a formula (12), so that the formula (11) may be expressed as a formula (13).

$$Ra = Rs/(N-M) \quad ---------- \quad (12)$$

$$Rm = Ra \times N \quad ------------- \quad (13)$$

The radiation exposure dose obtaining method described above may be provided as a program for causing a computer to perform the method.

A fourth radiation exposure dose obtaining apparatus of the present invention includes: a radiation exposure dose obtaining unit that obtains, based on a radiation image signal of each frame detected by a radiation image detector through continuous radiological imaging of a subject to be injected with a contrast agent, a radiation exposure dose of the subject due to the continuous radiological imaging; and a contrast agent frame identification unit that identifies a frame which includes an image signal representing an image of the contrast agent as a contrast agent frame from the radiation image signal of each frame, in which the radiation exposure dose obtaining unit obtains, for the radiation exposure dose of the contrast agent frame, a value corrected by linear interpolation using a radiation exposure value of a frame other than the contrast agent frame adjacent to the contrast agent frame on the front side and a radiation exposure value of a frame other than the contrast agent frame adjacent to the contrast agent frame on the back side.

A fourth radiation exposure dose obtaining method of the present invention obtains, based on a radiation image signal of each frame detected by a radiation image through continuous radiological imaging of a subject to be injected with a contrast agent, the method comprising the steps of; identifying a frame which includes an image signal representing an image of the contrast agent as a contrast agent frame from the radiation image signal of each frame; and obtaining, for the radiation exposure dose of the contrast agent frame, a value corrected by linear interpolation using a radiation exposure value of a frame other than the contrast agent frame adjacent to the contrast agent frame on the front side and a radiation exposure value of a frame other than the contrast agent frame adjacent to the contrast agent frame on the back side.

In the radiation exposure dose obtaining apparatus and method described above, the term "a frame other than the contrast agent frame adjacent to the contrast agent frame on the front side" as used herein refers to a frame within a several frames before and after a frame other than the contrast agent frame immediately preceding the contrast agent frame as the reference, and the term "a frame other than the contrast agent frame adjacent to the contrast agent frame on the back side" as used herein refers to a frame within a several frames before and after a frame other than the contrast agent frame immediately following the contrast agent frame as the reference.

In the radiation exposure dose obtaining apparatus and method described above, a sufficient correction effect may be obtained by a correction through linear interpolation using a frame other than a contrast agent frame immediately preceding the contrast agent frame and a frame other than the contrast agent frame immediately following the contrast agent frame. In the case where the identification accuracy is not perfect, in view of the frame rate at the time of imaging, contrast agent frame identification method, and actual imaging state the frame to be used by the linear interpolation may be changed from the frame at the reference position (immediately preceding or following the contrast agent frame) to a frame within a several frames before or after the frame at the reference position.

For example, in the case where a method in which a frame obtained during injection of a contrast agent is identified as a contrast agent frame, the contrast agent may possibly remain in the human body for a while after the injection of the contrast agent is ended. Thus, the frame on the back side of the contrast agent frame used for the linear interpolation may be changed to a frame further on the back side by the number of frames corresponding to the residual time with reference to the frame other than the contrast agent frame immediately following the contrast agent frame.

It is preferable that the radiation exposure dose is obtained based on an E.I. (Exposure Index) of the radiation image signal, but any value other than this may also be used as long as it serves as an index for determining how much of radiation is detected in each frame, such as an average value of signal values in each frame, a value calculated by considering the histogram, or the like. The value calculated based on the E.I. may be the value of E.I. itself or a value obtained by making a certain correction to the E.I.

The radiation exposure dose obtaining method described above may be provided as a program for causing a computer to perform the method.

According to the first radiation exposure dose obtaining apparatus, method, and program of the present invention, in the case where a radiation exposure dose of a subject is obtained, based on a radiation image signal of each frame detected by a radiation image detector through continuous radiological imaging of the subject, due to the continuous radiological imaging, an area or areas corresponding to a region of the subject is set in an image represented by the radiation image signal and the radiation exposure dose is obtained for each set area. In the case where disease risk management is performed for a patient, this allows more appropriate management considering different influences of radiation for each region of the body.

If an arrangement is adopted in which a frame which includes an image signal representing an image of a contrast agent injected into the subject as a contrast agent frame from the radiation image signal of each frame, and a correction is performed on a radiation exposure dose for the contrast agent frame to bring the radiation exposure dose close to an actual value, a more accurate radiation exposure dose of the subject may be obtained.

Further, if the radiation exposure dose is obtained based on an E.I. (Exposure Index) of the radiation image signal, the value may reflect the actual radiation exposure dose of the subject and a further accurate radiation exposure dose may be obtained.

According to the second radiation exposure dose obtaining apparatus, method, and program of the present invention, in the case where a radiation exposure dose of a subject is obtained, based on a radiation moving picture signal, which is a collection of a radiation image signal of each frame detected by a radiation image detector through continuous radiological imaging of the subject, a radiation exposure dose of the subject due to the continuous radiological imaging, a signal evaluation value that serves as an index for determining how much of radiation is detected over the entire radiation moving picture signal is calculated by a formula (1) capable of performing the calculation without using information of a frame which includes an error factor and a radiation exposure dose of the subject throughout the continuous radiological imaging is obtained based on the calculated signal evaluation value of the radiation moving picture signal. This allows an accurate radiation exposure dose to be obtained. Further, this requires only a simple calculation and the processing load may be reduced.

$$Rm = Rf \times N \quad \text{------------} \quad (1)$$

Further, the use of a maximum signal evaluation value among those calculated for each radiation image signal as the signal evaluation value of the radiation image signal of a predetermined frame may eliminate the concern that the radiation exposure dose of the subject is underestimated.

Further, as it is often the case that an error factor in obtaining a radiation exposure dose, such as contrast agent injection into a subject and the like, is not generally included right after the start of application of radiation to a subject, so that if the signal evaluation value of the radiation image signal of the first frame of the radiation moving picture signal is used as the signal evaluation value, a frame deemed not to include an error factor may be identified easily.

Further, if a value calculated based on an E.I. (Exposure Index) is used as the signal evaluation value of radiation image signal of a predetermined one frame, the value may reflect the actual radiation exposure dose of the subject and a further accurate radiation exposure dose may be obtained.

According to the third radiation exposure dose obtaining apparatus, method, and program of the present invention, in the case where a radiation exposure dose of a subject to be injected with a contrast agent is obtained, based on a radiation image signal of each frame detected by a radiation image detector through continuous radiological imaging of the subject, a radiation exposure dose of the subject due to the continuous radiological imaging, a frame which includes an image signal representing an image of the contrast agent as a contrast agent frame from the radiation image signal of each frame, a signal evaluation value of a radiation moving picture image, which is a collection of the radiation image signal of each frame, is calculated by a formula (11) that dose not use information of a contrast agent frame, and a radiation exposure dose of the subject throughout the continuous radiological imaging is obtained based on the calculated signal evaluation value of the radiation moving picture signal. This allows a more accurate radiation expose dose of the subject which takes into account the inclusion of an image of a contrast agent in the radiation image to be obtained.

$$Rm = Rs \times N/(N-M) \ \text{----------} \ (11)$$

Further, if a value calculated based on an E.I. (Exposure Index) is used as a signal evaluation value of the radiation image signal of each frame required for the calculation of the formula (11), the value may reflect the actual radiation exposure dose of the subject and a further accurate radiation exposure dose may be obtained.

According to the fourth radiation exposure dose obtaining apparatus, method, and program of the present invention, in the case where a radiation exposure dose of a subject to be injected with a contrast agent is obtained, based on a radiation image signal of each frame detected by a radiation image detector through continuous radiological imaging of the subject, a radiation exposure dose of the subject due to the continuous radiological imaging, a frame which includes an image signal representing an image of the contrast agent is identified as a contrast agent frame from the radiation image signal of each frame and, for the radiation exposure dose of the contrast agent frame, a value corrected by linear interpolation using a radiation exposure value of a frame other than the contrast agent frame adjacent to the contrast agent frame on the front side and a radiation exposure value of a frame other than the contrast agent frame adjacent to the contrast agent frame on the back side is used. This allows a more accurate radiation expose dose of the subject which takes into account the inclusion of an image of a contrast agent in the radiation image to be obtained.

Further, if a radiation exposure dose is obtained based on an E.I. (Exposure Index) of radiation image signal, the value may reflect the actual radiation exposure dose of the subject and a further accurate radiation exposure dose may be obtained.

Brief Description of Drawings

Figure 1 is a block diagram of a radiation image capturing system that uses an embodiment of a first radiation exposure dose obtaining apparatus of the present invention, illustrating an overall schematic configuration thereof.
Figure 2 is a flowchart illustrating an operation of the radiation image capturing system that uses an embodiment of the radiation exposure dose obtaining apparatus described above.
Figure 3 is a drawing for explaining an example radiation image and a region setting procedure (1).
Figure 4 illustrates a variation in the radiation exposure dose for each region.
Figure 5 is a drawing for explaining an example radiation image and a region setting procedure (2).
Figure 6 illustrates the DICOM saving format.
Figure 7 is a block diagram of a radiation image capturing system that uses an embodiment of a second radiation exposure dose obtaining apparatus of the present invention, illustrating an overall schematic configuration thereof.
Figure 8 is a flowchart illustrating an operation of the radiation image capturing system that uses an embodiment of the radiation exposure dose obtaining apparatus described above.
Figure 9 shows a timing chart illustrating the relationship among the radiation emission timing, charge accumulation timing in a radiation image detector, and contrast agent detection signal, and a graph representing radiation exposure doses calculated based on radiation image signal of each frame.
Figure 10 is a block diagram of a radiation image capturing system that uses an embodiment of a third radiation exposure dose obtaining apparatus of the present invention, illustrating an overall schematic configuration thereof.

Figure 11 is a flowchart illustrating an operation of the radiation image capturing system that uses an embodiment of the radiation exposure dose obtaining apparatus described above.

Figure 12 shows a timing chart illustrating the relationship among the radiation emission timing, charge accumulation timing in a radiation image detector, and contrast agent detection signal, and a graph representing radiation exposure doses calculated based on a radiation image signals of each frame.

Figure 13 is a block diagram of a radiation image capturing system that uses an embodiment of a fourth radiation exposure dose obtaining apparatus of the present invention, illustrating an overall schematic configuration thereof.

Figure 14 is a flowchart illustrating an operation of the radiation image capturing system that uses an embodiment of the radiation exposure dose obtaining apparatus described above.

Figure 15 shows a timing chart illustrating the relationship among the radiation emission timing, charge accumulation timing in a radiation image detector, and contrast agent detection signal, and a graph representing radiation exposure doses calculated based on radiation image signals of each frame.

Best Mode for Carrying Out the Invention

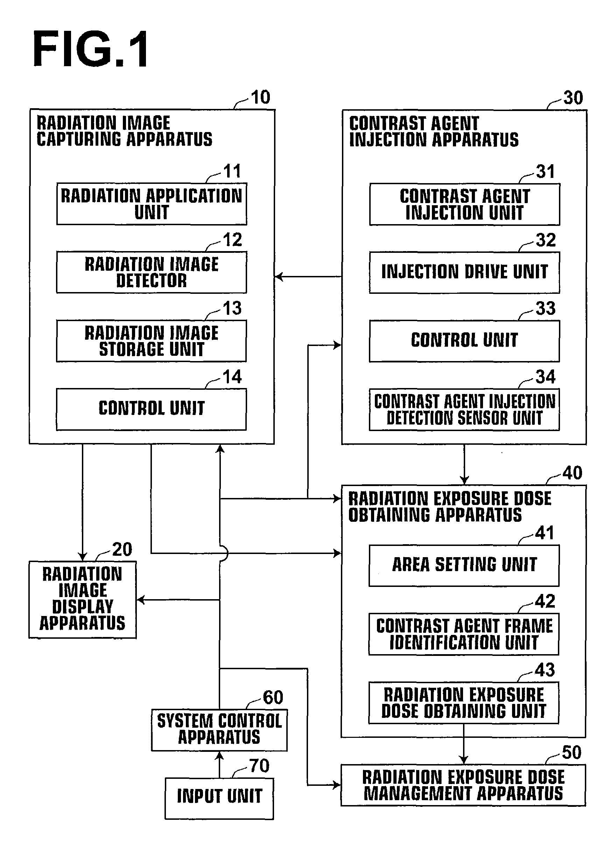

Hereinafter, an embodiment of a radiation image capturing system that uses an embodiment of the first radiation exposure dose obtaining apparatus of the present invention will be described with reference to the accompanying drawings. Figure 1 is a block diagram of the radiation image capturing system of the present embodiment, illustrating an overall schematic configuration thereof.

As illustrated in Figure 1, the radiation image capturing system of the present embodiment includes a radiation image capturing apparatus 10 that captures a fluoroscopic image (moving picture) of a patient, a radiation image display apparatus 20 that displays the fluoroscopic image captured by the radiation image capturing apparatus 10, a contrast agent injection apparatus 30 that injects a contrast agent into a blood vessel or a lymph vessel of the patient, a radiation exposure dose obtaining apparatus 40 that obtains an exposure dose of the patient based on an image signal of the fluoroscopic image captured by the radiation image capturing apparatus 10, a radiation exposure dose management apparatus 50 that stores and manages the exposure dose of each patient obtained by the radiation exposure dose obtaining apparatus 40, and a system control apparatus 60 that performs overall control of the radiation image capturing system.

As illustrated in Figure 1, the radiation image capturing apparatus 10 includes a radiation application unit 11 that has an X-ray tube, an aperture stop, and the like and applies radiation emitted from the x-ray tube and passed through the aperture stop to a patient, a radiation image detector 12 that detects radiation transmitted through the patient and outputs a radiation image signal representing a radiation image of the patient, a radiation image storage unit 13 that stores the radiation image signal outputted from the radiation image detector 12, and a control unit 14 that performs overall control of the radiation image capturing apparatus 10.

The radiation image detector 12 allows repeated use for recording and reading of radiation images, and a so-called direct type radiation image detector that records a radiation image by generating and accumulating a charge by directly receiving radiation or a so-called indirect type radiation image detector that records a radiation image by converting radiation first to visible light and then converting the visible light to a charge and accumulating the charge may be used. As for the radiation image signal reading method for reading out the radiation image recorded by accumulating charges in the manner described above, a so-called TFT (thin film transistor) reading method in which a radiation image signal is read by ON/OFF switching thin film transistors and a so-called optical reading method in which a radiation image signal is read out by applying reading light are preferably used, but others may also be used.

The radiation image capturing apparatus 10 is used to capture a fluoroscopic image (moving picture) of a subject to be injected with a contrast agent by the contrast agent injection apparatus 30, and the control unit 14 controls the radiation application unit 11 and radiation image detector 12 such that the fluoroscopic image is captured. More specifically, the control unit 14 controls the radiation application unit 11 to apply radiation at a predetermined frame rate and the radiation image detector 12 to perform recording and reading of a radiation image by the application of the radiation. Then, the radiation image signal of each frame outputted from the radiation image detector 12 is sequentially stored in the radiation image storage unit 13. Further, the control unit 14 of the present embodiment appends information indicating whether or not an image signal representing an image of the contrast agent is included in the radiation image signal of each frame when storing the radiation image signal of each frame in the radiation image storage unit 13, the operation of which will be described later in detail.

As for the structure of the radiation image capturing apparatus 10, a structure in which image capturing is performed with the patient being in the upright position or in the lateral position may be employed. Further, a structure that allows image capturing with the patient being both in upright position and lateral position may be employed.

The radiation image display apparatus 20 generates a display control signal by performing predetermined processing on the radiation image signal of each frame read out from the radiation image storage unit 13 of the radiation image capturing apparatus 10 and displays a fluoroscopic image of the patient on the monitor based on the display control signal.

The contrast agent injection apparatus 30 automatically injects a contrast agent into a patient based on an instruction input from the user or based on a preset injection start timing of the contrast agent. More specifically, the contrast agent injection apparatus 30 includes a contrast agent injection unit 31 formed of, for example, a syringe and injects a contrast agent into a blood vessel of the patient or the like, an injection drive unit 32 which drives the injection operation of the contrast agent injection unit 31, a control unit 33 that controls the operation of the injection drive unit 32, and a contrast agent injection detection sensor unit 34 that detects whether or not the contrast agent is being injected into a blood vessel or the like of the patient from the contrast agent injection apparatus 30.

The injection drive unit 32 moves, for example, the piston of the syringe of the contrast agent injection unit 31 and is formed of a power means, such as an air pressure, motor, or hydraulic pressure.

The contrast agent injection detection sensor unit 34 includes a sensor for detecting that the contrast agent is discharged from the contrast agent injection unit 31. Then, the contrast agent injection detection sensor unit 34 outputs a contrast agent detection signal to the control unit 14 of the radiation image capturing apparatus 10.

The radiation exposure dose obtaining apparatus 40 includes an area setting unit 41 that sets one or more areas corresponding to a body region or regions of a patient in an image represented by the radiation image signals of each frame read out from the radiation image storage unit 13 of the radiation image capturing apparatus 10, a contrast agent frame identification unit 42 that identifies a radiation image signal of a frame that includes an image signal of an image of a contrast agent as a contrast agent frame from the radiation image signal of each frame, and a radiation exposure dose obtaining unit 43 that obtains a radiation exposure dose of the patient for each region set by the area setting unit 41 based on the radiation image signals of each frame. Operations of the area setting unit 41, the contrast agent frame identification unit 42, and the radiation exposure dose obtaining unit 43 will be described later in detail.

The radiation exposure dose management apparatus 50 stores the radiation exposure dose obtained by the radiation exposure dose obtaining apparatus 40 and manages the radiation exposure dose of each patient.

The system control apparatus 60 outputs control signals to the radiation image capturing apparatus 10, the radiation image display apparatus 20, the contrast agent injection apparatus 30, the radiation exposure dose obtaining apparatus 40, and the radiation exposure dose management apparatus 50 to control the operations thereof and at the same time performs input/output signal control between these apparatuses. The system control apparatus 60 is provided with an input unit 70 which receives an instruction input and other inputs, such as image capturing conditions, patient ID information and the like, from the user. The input information received at the input unit 70 is outputted to each apparatus by the system control apparatus 60 as required.

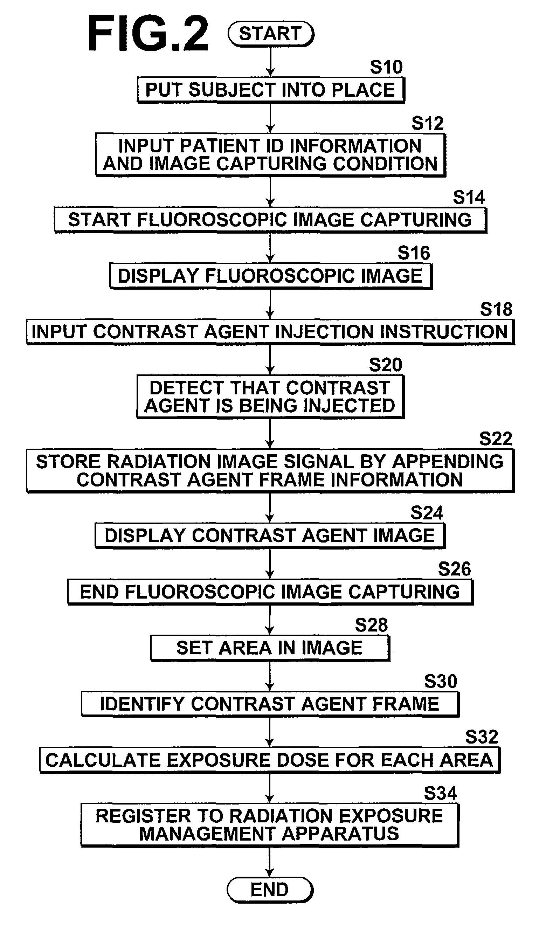

An operation of the radiation image capturing system of the present embodiment will now be described with reference to the flowchart of Figure 2.

First, a patient, the subject of the image capturing, is placed on an image capturing platform or the like provided in the radiation image capturing apparatus 10 and the patient is put into position (S10).

Then, ID information of the image capturing target patient and an image capturing condition are inputted by the user using the input unit 70 and the patient ID information is registered in the radiation exposure dose management apparatus 50 while the image capturing condition is set to the control unit 14 of the radiation image capturing apparatus 10 (S12). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user using the input unit 70 and in response to the input, a control signal is outputted from the system control apparatus 60 to the radiation image capturing apparatus 10 to capture a fluoroscopic image, and the radiation image capturing apparatus 10 starts fluoroscopic image capturing according to the inputted control signal (S14).

More specifically, the X-ray tube of the radiation application unit 11 is controlled based on the inputted imaging condition and a predetermined dose of radiation is applied toward the patient intermittently at a predetermined frame rate.

Then, radiation transmitted through the patient is applied to the radiation image detector 12 and subjected to a photoelectrical conversion in the radiation image detector 12 and accumulated therein as a charge signal.

Thereafter, each time the application of radiation for each frame is ended, the charge signal accumulated in the radiation image detector 12 is read out by the control unit 14, then converted to a digital signal by an A/D converter (not shown), and stored in the radiation image storage unit 13.

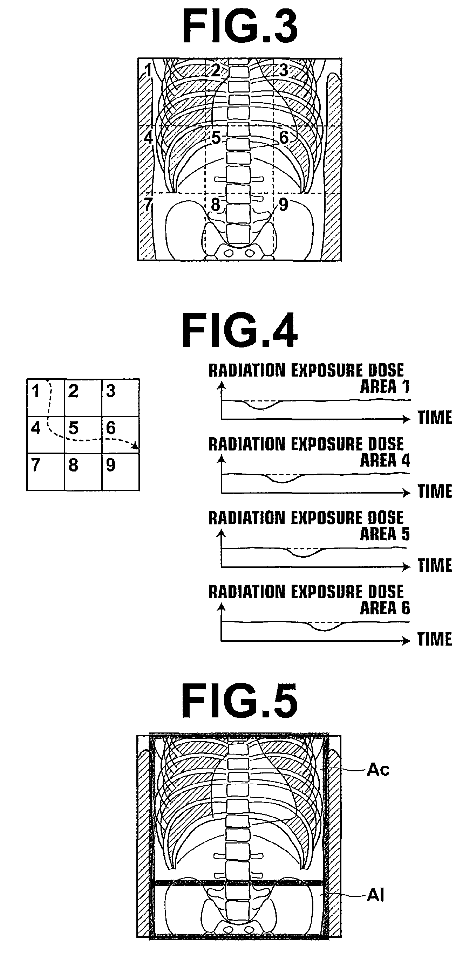

Figure 3 is a timing chart illustrating the application timing of radiation from the X-ray tube of the radiation application unit 11 and the charge accumulation timing of the radiation image detector 12. The period during which no charge accumulation is performed in the radiation image detector 12 (accumulation OFF period) is a period for reading out a charge signal from the radiation image detector 12.

The repeated performance of radiation application by the X-ray tube and the radiation image recording and reading in the radiation image detector 12 at a predetermined frame rate in the manner described above causes the radiation image signal of each frame to be sequentially stored in the radiation image storage unit Then, the radiation image signal of each frame stored in the radiation image storage apparatus 13 is sequentially read out and outputted to the radiation image display apparatus 20. The radiation image display apparatus 20 sequentially generates a display control signal based on the inputted radiation image signal of each frame and sequentially outputs the display control signal to the monitor to display a fluoroscopic image of the patient as a moving picture (S16). It is assumed here that the radiation image signal of each frame stored in the radiation image storage unit 13 will remain un-erased in the radiation image storage unit 13 after being read out.

Here, if the user desires to observe a contrast agent image, such as a blood vessel or the like, while the fluoroscopic image capturing and display are performed in the manner described above, a contrast agent injection instruction is inputted using the input unit 70 (S18).

When the contrast agent injection instruction is inputted at the input unit 70, a control signal is outputted from the system control apparatus 60 to the contrast agent injection apparatus 30. The contrast agent injection apparatus 30 starts contrast agent injection based on the inputted control signal. More specifically, the contrast agent injection unit 31 is driven by the injection drive unit 32 and a predetermined amount of contrast agent is discharged from the contrast agent injection unit 31.

Here, the contrast agent injection detection sensor unit 34 provided in the contrast agent injection apparatus 30 detects that the contrast agent is discharged and a detection signal is outputted to the control unit 14 of the radiation image capturing apparatus 10 (S20). When the contrast agent detection signal is inputted, the control unit 14 of the radiation image capturing apparatus 10 appends to a radiation image signal of a frame captured while the contrast agent detection signal is inputted information indicating that it includes an image signal representing an image of the contrast agent (hereinafter, referred to as "contrast agent frame information") as header information, and stores the radiation image signal in the radiation image storage unit 13 with the header information (S22). More specifically, if the contrast agent detection signal is inputted to the radiation image capturing apparatus 10 in the timing illustrated in Figure 3, the contrast agent frame information is appended to the radiation image signals of frames F1 and F2 captured while the contrast agent is injected and stored in the radiation image storage unit 13.

Then, the radiation image signals captured while the contrast agent is injected are also read out from the radiation image storage unit 13 and sequentially outputted to the radiation image display apparatus 20. The radiation image display apparatus 20 sequentially generates display control signals based on the inputted radiation image signals, and displays radiation images including a contrast agent image on the monitor based on the display control signals (S24).

Then, after the discharge of the predetermined amount of contrast agent from the contrast agent injection apparatus 30 is completed and the contrast agent detection signal is not detected any more in the contrast agent injection detection sensor unit 34, radiation image signals captured are stored in the radiation image storage unit 13 without the contrast agent frame information being appended, and the radiation image signal of each frame is read out from the radiation image storage unit 13 in the manner described above and a fluoroscopic image is displayed on the radiation image display apparatus 20.

Thereafter, when an instruction to end the fluoroscopic image capturing is inputted by the user through the input unit 70, the system control apparatus 60 outputs a control signal to the radiation image capturing apparatus 10 to end the fluoroscopic image capturing, and the radiation image capturing apparatus 10 ends the fluoroscopic image capturing in response to the inputted control signal (S26).

When the fluoroscopic image capturing described above is ended, the system control apparatus 60 reads out radiation image signals of all frames stored in the radiation image storage unit 13 of the radiation image capturing apparatus 10 and inputs the radiation image signals to the radiation exposure dose obtaining apparatus 40.

Then, first, the area setting unit 41 sets a region in an image represented by the radiation image signal of each frame corresponding to a body region of the patient (S28). More specifically, the image represented by the radiation image signal is segmented into 9 segmented areas in square mesh, as illustrated in Figure 3, and each segmented area is associated with a body region of the patient. If the image shown in Figure 3 is taken as an example, the division is made such that the segmented areas 1 to 6 are categorized as the chest region and segmented areas 7 to 9 are categorized as the lumbar region.

As for the method of associating each segmented area with a body region of the patient, a method in which an association instruction is received from the user to arbitrarily associate each segmented area with a body region of the patient or a method in which each segmented area is automatically associated with a body region of the patient based on the information, such as the patient ID information, imaged region, image capturing conditions, may be used. Further, the size, shape, and the number of the segmented areas are not limited to those described above and, for example, in the case where a strict management is required, the size of the segmented areas may be reduced while the number of the segmented areas may be increased.

Further, instead of segmenting the image represented by the radiation image signal into a plurality of segmented areas in advance, an area corresponding to a region of the subject is directly identified in the image represented by the radiation image signal using a known image recognition technology and the identified region may be associated with a region of the subject. By way of example, in the case of the image shown in Figure 5, an area Ac and an area Al corresponding respectively to the chest region and the lumbar region of the subject are identified in the image using a known image recognition technology and the area Ac may be treated as the chest region while the area Al may be treated as the lumbar region. Further, any other mode may be used.

Next, the contrast agent frame identification unit 42 identifies a frame which includes an image signal representing an image of a contrast agent as the contrast agent frame for each segmented area segmented by the area setting unit 41 (S30), and the radiation exposure dose obtaining unit 43 calculates the radiation exposure dose received by the patient during capturing of each frame of the fluoroscopic image based on the radiation image signals of each frame for each segmented area segmented by the area setting unit 41 (S32).

More specifically, an E.I. (Exposure Index) is calculated as a signal evaluation value of each segmented area based on the radiation image signal of each frame, and the radiation exposure dose is obtained based on the E.I.

The E.I. is calculated in the following manner. First, a predetermined calculation area is set in a radiation image of each segmented area. The calculation area may be, for example, the entire area of the radiation image, an area arbitrarily set by the user, an area defined based on the imaged region information, or an area within 10% of the image size from the center of the radiation image. Otherwise, an area except for the so-called direct exposure area or an area of 90% of the entire density width from the central density of the radiation image may be employed. Alternatively, a specific calculation area may be set by combining the aforementioned conditions.

Next, a representative value V of the calculation area set in the above is calculated. As for the representative value V, the density value itself of the radiation image or a statistical characteristic value obtained by modifying the density value itself with the average value of the entire density values, median value, mode value, or trimmed mean value may be employed. Then, based on the representative value V, the E.I. of each frame is calculated by the formula given below:

$$\mathrm{E.I.} = C_0 \times g\,(V),$$

where:

$g(V)$: reverse calibration function; and
$C_0$: $100 \cdot Gy$ (constant).

The $g(V)$ is a function defined based on a radiation image obtained with the radiation quality of RQA5. The representative value V differs in magnitude due to difference in sensitivity arising from difference in scintillator used in the radiation image detector or difference in the setting method of the calculation area or the calculation method of the representative value V and $g(V)$ is a function to normalize the differences. That is, if RQA5 radiation is received by any type of radiation image detector by the same radiation dose, the E.I. will become nearly the same value.

The radiation exposure dose received by the patient in each segmented area during imaging of each frame is calculated by the radiation exposure dose obtaining unit 43 using the E.I, calculated in the manner described above. As for the method of obtaining the radiation exposure dose using the E.I., for example, a function which defines these relationships or a lookup table may be set in advance.

Here, in the case where a contrast agent is injected and a contrast agent image is captured in the middle of the fluoroscopic image capturing as described above, the radiation applied to the patient is absorbed by the contrast agent before reaching the radiation image detector 12, so that if the radiation exposure dose is calculated based on the radiation image signal of the contrast agent frame, the calculated dose becomes smaller than that actually received by the patient.

The graph on the right in Figure 4 illustrates the radiation exposure doses obtained in the manner described above for each frame in each segmented area connected by a line and the horizontal axis of the graph indicates the frame number from the start of imaging. For example, in the case where the amount of radiation exposure is calculated based on the radiation image signal shown in the upper most graph on the right in Figure 4, the radiation exposure dose in the area where a contrast agent is imaged (depressed area in the graph) will become smaller than the actual value (estimated value indicated by the dotted line in the graph).

Further, as indicated by the dotted line in the drawing on the left in Figure 4, the contrast agent injected into the human body may possibly move with time. In this case, as illustrated in the graph on the right in Figure 4, the time or length of the portion where the radiation exposure dose becomes a smaller value affected by the contrast agent differs depending on the segmented area, so that a frame which includes an image signal representing an image of a contrast agent is identified in the contrast agent frame identification unit 42 for each segmented area and correction is performed in the radiation exposure dose obtaining unit 43 to bring the smaller value closer to the actual value of radiation exposure dose for the contrast agent frame.

In the present embodiment, the contrast agent frame identification is performed such that a frame whose radiation exposure dose is dipped more than or equal to a predetermined value with respect to the average of the radiation exposure doses of the entire graph is identified as a contrast agent frame. For the correction of the radiation exposure dose, a value corrected by a linear interpolation using a radiation exposure dose of a frame other than a contrast agent frame immediately preceding the contrast agent frame and a radiation exposure dose of a frame other than a contrast agent frame immediately following the contrast agent frame is used for each segmented area.

Then, the radiation exposure dose obtaining unit 43 calculates a radiation exposure dose of the subject for each segmented area throughout the continuous radiological imaging by adding radiation exposure doses of all frames of each segmented area and, based on this, calculates a total radiation exposure dose for each body region of the patient. More specifically, as the segmented areas 1 to 6 are categorized as the chest region and the segmented areas 7 to 9 are categorized as the lumbar region in the present embodiment, the sum of the radiation exposure doses of segmented areas 1 to 6 is the total radiation exposure dose of the chest region while the sum of the radiation exposure doses of segmented areas 7 to 9 is the total radiation exposure dose of the lumbar region.

The total radiation exposure dose of each body region of the patient obtained in the radiation exposure dose obtaining unit 43 is inputted to the radiation exposure dose management apparatus 50, and the radiation exposure dose management apparatus 50 registers the total radiation exposure dose of each body region of the patient with the ID information of the patient inputted in advance (S34). Then, the radiation exposure dose management apparatus 50 displays the registered total radiation exposure dose of each body region of the patient with the ID information of the patient as required or calculates a cumulative radiation exposure dose from the past for a particular patient and displays a warning message if the cumulative radiation exposure dose is greater than a predetermined specified value.

When storing the total radiation exposure dose of each body region of the patient, the use of a standard format may result in improved convenience. As the standard protocol in the medical image systems is DICOM (Digital Imaging and COmmunication in Medicine), storage in a format based on the DICOM is preferable. As for the saving format of the radiation dose in fluoroscopic image capturing, the study and proposal have been made by the Working Group, but the saving format in association with body regions of a patient is not studied. As such, an example format which takes account the association of body regions of a patient with the saving format proposed by the working Group will be described below.

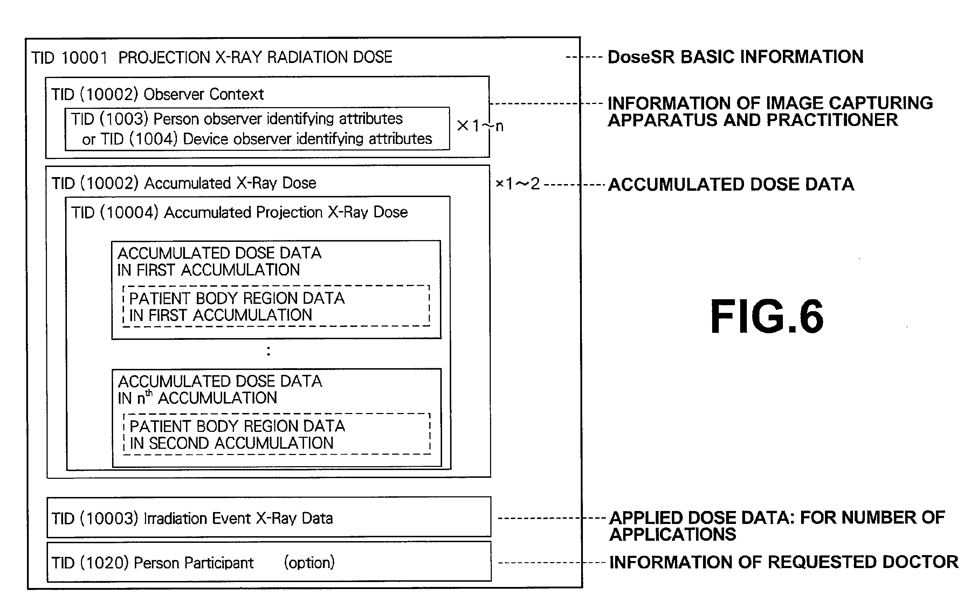

Figure 6 illustrates the current DICOM saving format for radiation dose of a fluoroscopic image. Note that, in Figure 6, the solid line illustrates the contents proposed by the Working Group while the dotted line illustrates the contents which take account the saving with body regions of a patient being associated according to the present embodiment. The saving format of the radiation dose in fluoroscopic image capturing has a hierarchical structure and the saving of accumulated dose (detected radiation dose at the radiation image detector) and the saving of applied dose (applied radiation dose at the radiation application unit) are proposed. In Figure 6, it is assumed that n times of accumulation are performed, and a format of saving a corresponding accumulated dose for each time of accumulation is illustrated, but a format in which the sum of accumulated doses corresponding to each of a plurality of times of accumulation is saved is also allowed. The same applies to the applied dose.

As the radiation exposure dose of the present embodiment corresponds to the accumulated dose, it is saved under the hierarchy of "TID (10004) Accumulated Application X-Ray Dose (accumulated dose) " and a body region of the patient is also associated therewith and saved. The saving format of body regions may be a text format, like "abdomen" if the region is abdomen. In the case where the sum of accumulated doses of a plurality of times of accumulation for the plurality of times of accumulation is saved, it is highly likely that the body region of the patient extends to a plurality of regions and the plurality of body regions may be saved in a list format with "and", like "chest and abdomen" if the regions are chest and abdomen.

In the case where the radiation application unit does not have any means for measuring the applied dose, no data exists under the hierarchy of "TID (10003) Irradiation Event X-Ray Data (applied dose) " and it may cause inconvenience to the subsequent processing. In such a case, instead of the applied dose, radiation exposure dose (accumulated dose) may be saved under the hierarchy of "TID (10003) Irradiation Event X-Ray Data (applied dose)". In this case, the saving may be performed in the same manner as in the saving format of the accumulated dose described above.

According to the radiation image capturing system of the present embodiment, when obtaining a radiation exposure dose of a subject during the continuous radiological imaging based on a radiation image signal of each frame detected by the radiation image detector through the continuous radiological imaging of the subject, one or more areas corresponding to a region or regions of the subject are set in an image represented by the radiation image signal, and a radiation exposure dose is obtained for each area. When performing risk management of diseases for patients, this allows more appropriate management which takes into account different impacts of radiation on different body regions to be performed.

So far, a preferred embodiment of the first radiation exposure dose obtaining apparatus of the present invention has been described, but the first radiation exposure dose obtaining apparatus of the present invention is not limited to the aforementioned embodiment. For example, a contrast agent frame may be identified by any method other than the aforementioned method, such as a method which uses contrast agent frame information to identify, for example, a frame having contrast agent frame information appended thereto is identified as a contrast agent frame or a frame after a predetermined time from the frame having contrast agent frame information appended thereto is indentified as a contrast agent frame by taking into account the traveling time of the contrast agent within the body.

Further, in the radiation image capturing system described above, a radiation exposure dose of a patient during a contrast agent frame is captured through linear interpolation of radiation exposure doses calculated based on radiation image signals of immediately preceding and immediately following frames of the contrast agent frame. But the method is not limited to this and any other method may be used. For example, a radiation exposure dose calculated based on the radiation image signal of the immediately preceding or immediately following frame may be employed directly as the radiation exposure dose during the contrast agent frame capturing, or a radiation exposure dose calculated based on the radiation image signal of another frame other than the contrast agent frame may be employed. Otherwise, a radiation exposure dose calculated based on the radiation image signal of a contrast agent frame may be corrected by adding a predetermined radiation exposure dose determined in advance thereto or by multiplying it with a given coefficient which is greater than 1.

Further, the radiation exposure dose obtaining apparatus is implemented as an independent apparatus in the embodiment described above, but it may be implemented in any other form, such as being incorporated in the other apparatus, such as the radiation image capturing apparatus, as a part thereof. More specifically, it may be provided in a console which includes the system control apparatus 60 or in the radiation image capturing apparatus 10. Further, in the case where the radiation image detector 12 is accommodated in a portable electronic cassette, and hardware of electronic circuits, such as LSI (Large Scale Integration), hardware of programmable electronic circuits, such as PLD (Programmable Logic Device) · FPGA (Field-Programmable Gate Array), and the like are accommodated in the electronic cassette, the identification of a contrast agent frame and acquisition of radiation exposure dose may be performed by the hardware. Such arrangement allows pursuit of more real time implementation.

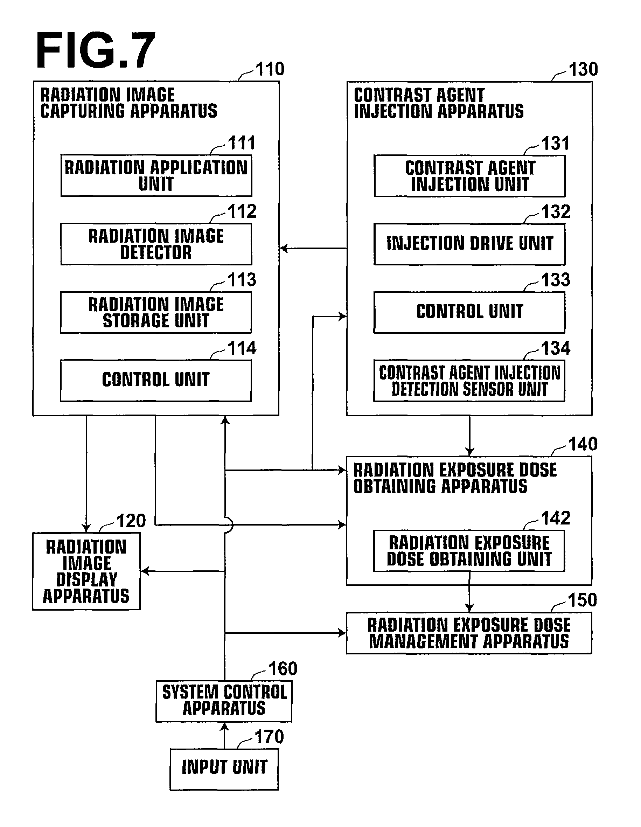

An embodiment of a radiation image capturing system that uses an embodiment of the second radiation exposure dose obtaining apparatus of the present invention will now be described with reference to the accompanying drawings. Figure 7 is a block diagram of the radiation image capturing system of the present embodiment, illustrating an overall schematic configuration thereof.

As illustrated in Figure 7, the radiation image capturing system of the present embodiment includes a radiation image capturing apparatus 110 which captures fluoroscopic image (moving picture) of a patient, a radiation image display apparatus 120 which displays the fluoroscopic image captured by the radiation image capturing apparatus 110, a contrast agent injection apparatus 130 which injects a contrast agent into a blood vessel or a lymph vessel of the patient, a radiation exposure dose obtaining apparatus 140 which obtains an exposure dose of the patient based on an image signal of the fluoroscopic image captured by the radiation image capturing apparatus 110, a radiation exposure dose management apparatus 150 which stores and manages the exposure dose of each patient obtained by the radiation exposure dose obtaining apparatus 140, a system control apparatus 160 which performs overall control of the radiation image capturing system.

As illustrated in Figure 7, the radiation image capturing apparatus 110 includes a radiation application unit 111 that has an X-ray tube, an aperture stop, and the like and applies radiation emitted from the x-ray tube and passed through the aperture stop to a patient, a radiation image detector 112 that detects radiation transmitted through the patient and outputs a radiation image signal representing a radiation image of the patient, a radiation image storage unit 113 that stores the radiation image signal outputted from the radiation image detector 112, and a control unit 114 that performs overall control of the radiation image capturing apparatus 110.

The radiation image detector 112 allows repeated use for recording and reading of radiation images, and a so-called direct type radiation image detector that records a radiation image by generating and accumulating a charge by directly receiving radiation or a so-called indirect type radiation image detector that records a radiation image by converting radiation first to visible light and then converting the visible light to a charge and accumulating the charge may be used. As for the radiation image signal reading method for reading out the radiation image recorded by accumulating charges in the manner described above, a so-called TFT (thin film transistor) reading method in which a radiation image signal is read by ON/OFF switching thin film transistors and a so-called optical reading method in which a radiation image signal is read out by applying reading light are preferably used, but others may also be used.

The radiation image capturing apparatus 110 is used to capture a fluoroscopic image (moving picture) of a subject to be injected with a contrast agent by the contrast agent injection apparatus 130, and the control unit 114 controls the radiation application unit 111 and radiation image detector 112 such that the fluoroscopic image is captured. More specifically, the control unit 114 controls the radiation application unit 111 to apply radiation at a predetermined frame rate and the radiation image detector 112 to perform recording and reading of a radiation image by the application of the radiation. Then, the radiation image signals of each frame outputted from the radiation image detector 112 are sequentially stored in the radiation image storage unit 113. Further, the control unit 114 of the present embodiment appends information indicating whether or not an image signal representing an image of the contrast agent is included in the radiation image signals of each frame when storing the radiation image signal of each frame in the radiation image storage unit 113, the operation of which will be described later in detail.

As for the structure of the radiation image capturing apparatus 110, a structure in which image capturing is performed with the patient being in the upright position or in the lateral position may be employed. Further, a structure that allows image capturing with the patient being both in upright position and lateral position may be employed.

The radiation image display apparatus 120 generates a display control signal by performing predetermined processing on the radiation image signals of each frame read out from the radiation image storage unit 113 of the radiation image capturing apparatus 110 and displays a fluoroscopic image of the patient on the monitor based on the display control signal.

The contrast agent injection apparatus 130 automatically injects a contrast agent into a patient based on an instruction input from the user or based on a preset injection start timing of the contrast agent. More specifically, the contrast agent injection apparatus 130 includes a contrast agent injection unit 131 formed of, for example, a syringe and injects a contrast agent into a blood vessel of the patient or the like, an injection drive unit 132 which drives the injection operation of the contrast agent injection unit 131, a control unit 133 that controls the operation of the injection drive unit 132, and a contrast agent injection detection sensor unit 134 that detects whether or not the contrast agent is being injected into a blood vessel or the like of the patient from the contrast agent injection apparatus 130.

The injection drive unit 132 moves, for example, the piston of the syringe of the contrast agent injection unit 131 and is formed of a power means, such as an air pressure, motor, or hydraulic pressure.

The contrast agent injection detection sensor unit 134 includes a sensor for detecting that the contrast agent is discharged from the contrast agent injection unit 131. Then, the contrast agent injection detection sensor unit 134 outputs a contrast agent detection signal to the control unit 114 of the radiation image capturing apparatus 110.

The radiation exposure dose obtaining apparatus 140 includes a radiation exposure dose obtaining unit 142 that obtains a radiation exposure dose of a patient based on a radiation moving picture signal which is a collection of radiation image signals of each frame read out from the radiation storage unit 113 of the radiation image capturing apparatus 110. The operation of the radiation exposure dose obtaining unit 142 will be described later in detail.

The radiation exposure dose management apparatus 150 stores the radiation exposure dose obtained by the radiation exposure dose obtaining apparatus 140 and manages the radiation exposure dose of each patient.

The system control apparatus 160 outputs control signals to the radiation image capturing apparatus 110, the radiation image display apparatus 120, the contrast agent injection apparatus 130, the radiation exposure dose obtaining apparatus 140, and the radiation exposure dose management apparatus 150 to control the operations thereof and at the same time performs input/output signal control between these apparatuses. The system control apparatus 160 is provided with an input unit 170 which receives an instruction input and other inputs, such as image capturing conditions, patient ID information and the like, from the user. The input information received by the input unit 170 is outputted to each apparatus by the system control apparatus 160 as required.

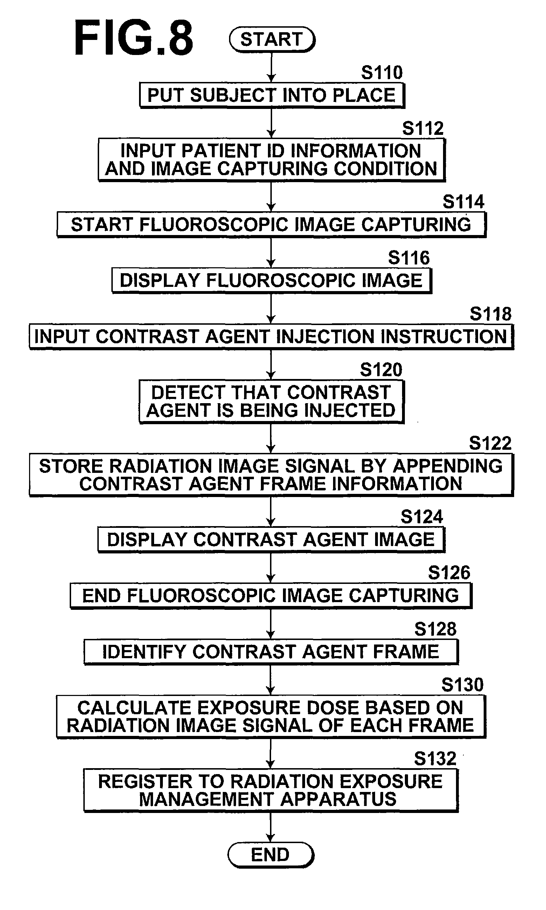

An operation of the radiation image capturing system of the present embodiment will now be described with reference to the flowchart of Figure 8.

First, a patient, the subject of the image capturing, is placed on an image capturing platform or the like provided in the radiation image capturing apparatus 110 and the patient is put into position (S110).

Then, ID information of the image capturing target patient and an image capturing condition are inputted by the user through the input unit 170 and the patient ID information is registered in the radiation exposure dose management apparatus 150 while the image capturing condition is set to the control unit 114 of the radiation image capturing apparatus 110 (S112). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user through the input unit 170 and in response to the input, a control signal is outputted from the system control apparatus 160 to the radiation image capturing apparatus 110 to capture a fluoroscopic image, and the radiation image capturing apparatus 110 starts fluoroscopic image capturing according to the inputted control signal (S114).

More specifically, the X-ray tube of the radiation application unit 111 is controlled based on the inputted imaging condition and a predetermined dose of radiation is applied toward the patient intermittently at a predetermined frame rate.

Then, radiation transmitted through the patient is applied to the radiation image detector 112 and subjected to a photoelectrical conversion in the radiation image detector 112 and accumulated therein as a charge signal.

Thereafter, each time the application of radiation for each frame is ended, the charge signal accumulated in the radiation image detector 112 is read out by the control unit 114, then converted to a digital signal by an A/D converter (not shown), and stored in the radiation image storage unit 113.

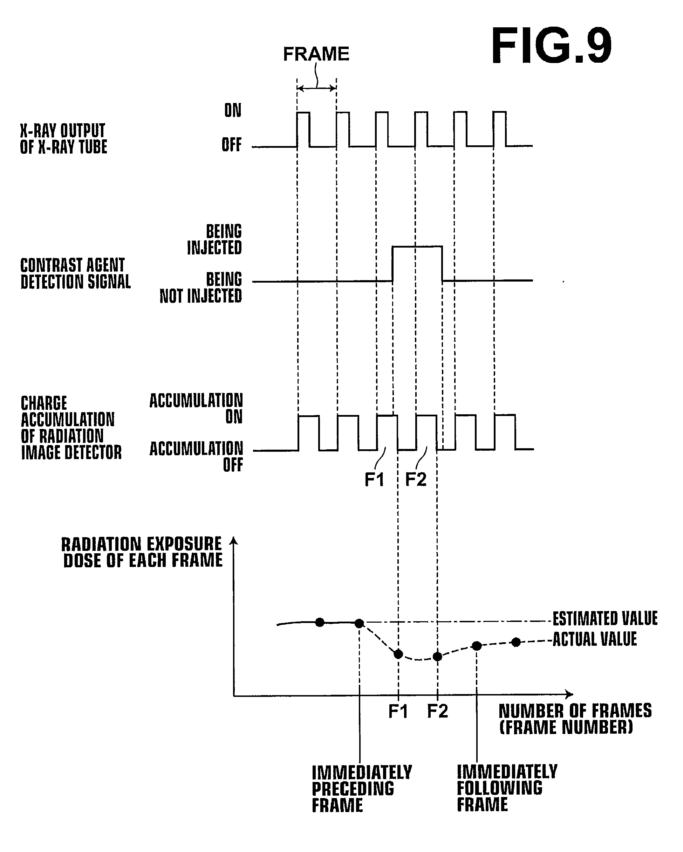

Figure 9 is a timing chart illustrating the application timing of radiation from the X-ray tube of the radiation application unit 111 and the charge accumulation timing of the radiation image detector 112. The period during which no charge accumulation is performed in the radiation image detector 112 (accumulation OFF period) is a period for reading out a charge signal from the radiation image detector 112.

The repeated performance of radiation application by the X-ray tube and the radiation image recording and reading in the radiation image detector 112 at a predetermined frame rate in the manner described above causes the radiation image signal of each frame to be sequentially stored in the radiation image storage unit 113.

Then, the radiation image signals of each frame stored in the radiation image storage apparatus 113 are sequentially read out and outputted to the radiation image display apparatus 120. The radiation image display apparatus 120 sequentially generates display control signals based on the inputted radiation image signals of each frame and sequentially outputs the display control signals to the monitor to display a fluoroscopic image of the patient as a moving picture (S116). It is assumed here that the radiation image signals with respect each frame stored in the radiation image storage unit 113 will remain un-erased in the radiation image storage unit 113 after being read out.

Here, if the user desires to observe a contrast agent image, such as a blood vessel or the like, while the fluoroscopic image capturing and display are performed in the manner described above, a contrast agent injection instruction is inputted through the input unit 170 (S118).

When the contrast agent injection instruction is inputted through the input unit 170, a control signal is outputted from the system control apparatus 160 to the contrast agent injection apparatus 130. The contrast agent injection apparatus 130 starts contrast agent injection based on the inputted control signal. More specifically, the contrast agent injection unit 131 is driven by the injection drive unit 132 and a predetermined amount of contrast agent is discharged from the contrast agent injection unit 131.

Here, the contrast agent injection detection sensor unit 134 provided in the contrast agent injection apparatus 130 detects that the contrast agent is discharged and a detection signal is outputted to the control unit 114 of the radiation image capturing apparatus 110 (S120). When the contrast agent detection signal is inputted, the control unit 114 of the radiation image capturing apparatus 110 appends to the radiation image signal of a frame captured while the contrast agent detection signal is inputted information indicating that it includes an image signal representing an image of the contrast agent(hereinafter, referred to as "contrast agent frame information") as header information, and stores the radiation image signal in the radiation image storage unit 113 with the header information (S122). More specifically, if the contrast agent detection signal is inputted to the radiation image capturing apparatus 110 in the timing illustrated in Figure 9, the contrast agent frame information is appended to the radiation image signals of frames F1 and F2 captured while the contrast agent is injected and stored in the radiation image storage unit 113.

Then, the radiation image signals captured while the contrast agent is injected are also read out from the radiation image storage unit 113 and sequentially outputted to the radiation image display apparatus 120. The radiation image display apparatus 120 sequentially generates display control signals based on the inputted radiation image signals, and displays radiation images including a contrast agent image on the monitor based on the display control signals (S124).

Then, after the discharge of the predetermined amount of contrast agent from the contrast agent injection apparatus 130 is completed and the contrast agent detection signal is not detected any more in the contrast agent injection detection sensor unit 134, radiation image signals captured are stored in the radiation image storage unit 113 without the contrast agent frame information being appended, and radiation image signals with respect each frame are read out from the radiation image storage unit 113 in the manner described above and a fluoroscopic image is displayed on the radiation image display apparatus 120.

Thereafter, when an instruction to end the fluoroscopic image capturing is inputted by the user through the input unit 170, the system control apparatus 160 outputs a control signal to the radiation image capturing apparatus 110 to end the fluoroscopic image capturing, and the radiation image capturing apparatus 110 ends the fluoroscopic image capturing in response to the inputted control signal (S126).

When the fluoroscopic image capturing described above is ended, the system control apparatus 160 reads out radiation image signals of all frames, i.e., the radiation moving picture signal, stored in the radiation image storage unit 113 of the radiation image capturing apparatus 110 and inputs the radiation image signals to the radiation exposure dose obtaining apparatus 140.

Then, first, a contrast agent frame identification unit 141 identifies a radiation image signal of a frame to which contrast agent frame information is appended from the radiation moving picture signal and identifies the frame as a contrast agent frame (S128).

Next, the radiation exposure dose obtaining unit 142 calculates the radiation exposure dose received by the patient during capturing of the fluoroscopic image based on radiation image signals of each frame (S130).

More specifically, an E.I. (Exposure Index) is calculated as a signal evaluation value Rf based on a radiation image signal of the first frame of the radiation moving picture signal.

The E.I. is calculated in the following manner. First, a predetermined calculation area is set in a radiation image of each frame. The calculation area may be, for example, the entire area of the radiation image, an area arbitrarily set by the user, an area defined based on the imaged region information, or an area within 10% of the image size from the center of the radiation image. Otherwise, an area except for the so-called direct exposure area which may be obtained based on, for example, a histogram of the radiation image or an area of 90% of the entire density width from the central density of the radiation image may be employed. Alternatively, a specific calculation area may be set by combining the aforementioned conditions.

Next, a representative value V of the calculation area set in the above is calculated. As for the representative value V, the density value itself of the radiation image or a statistical characteristic value obtained by modifying the density value itself with the average value of the entire density values, median value, mode value, or trimmed mean value may be employed. Then, based on the representative value V, the E.I. of each frame is calculated by the formula given below:

$$E.I. = C_0 \times g\ (V),$$

where:

g(V): reverse calibration function; and
$C_0$: 100·Gy (constant).

Here, g(V) is a function defined based on a radiation image obtained with the radiation quality of RQA5. The representative value V differs in magnitude due to difference in sensitivity arising from difference in scintillator used in the radiation image detector or difference in the setting method of the calculation area or the calculation method of the representative value V and g(V) is a function to normalize the differences. That is, if RQA5 radiation is received by any type of radiation image detector by the same radiation dose, the E.I. will become nearly the same value.

Next, an E.I. of the radiation moving picture signal (collection of radiation image signals of each frame) is calculated by the formula (1) and a radiation exposure dose of the subject during the continuous radiological imaging based on the calculated E.I. of the radiation moving picture signal. As for the method of obtaining the radiation exposure dose using the E.I., for example, a function which defines these relationships or a lookup table may be set in advance.

$$Rm = Rf \times N\ \text{----------------}\ (1)$$

where:

Rm: E.I. of the entire radiation moving picture signal (signal evaluation value of the entire radiation moving picture signal);
N: number of frames of the entire radiation moving picture signal; and
Rf: E.I. of the radiation image signal of the first frame of the radiation moving picture signal.

In the case where a contrast agent is injected and a contrast agent image is captured in the middle of the fluoroscopic image capturing as described above, the radiation applied to the patient is absorbed by the contrast agent before reaching the radiation image detector 112, as illustrated in the graph shown at the bottom of Figure 9. Therefore, if a radiation exposure dose is calculated for each frame based simply on a radiation image signal of each frame and a radiation exposure dose of the entire moving picture (radiation exposure dose of the subject due to the continuous radiological imaging) is obtained by adding radiation exposure doses of all the frames of the moving picture, the obtained radiation exposure dose becomes smaller than that actually received by the patient.

The graph at the bottom of Figure 9 illustrates the radiation exposure dose of each frame connected by a line and the horizontal axis of the graph indicates the frame number from the start of imaging. More specifically, if a radiation exposure dose is calculated based on the radiation image signals of frames F1, F2 which are deemed to have a contrast agent image illustrated in Figure 9, the calculated radiation exposure dose will become smaller than the actual value (estimated value indicated by the dash-dot line in the graph of Figure 9) .

Further, even in a frame deemed not to have a contrast agent image following the frames F1, F2, the signal value of the entire image is slightly varied due to a residual contrast agent in the subject and the radiation exposure dose becomes smaller than the actual value (estimated value indicated by the dash-dot line in the graph of Figure 9), though not so great as those of the frames F1, F2 deemed to have a contrast agent image due to the same reason as in the frames F1, F2. Note that shape of the graph is illustrated in an exaggerated manner than the actual variation in order to clearly indicate the nature of the variation.

In the present embodiment, however, the E.I. of the entire radiation moving picture signal is calculated by the formula (1) capable of performing the calculation without using information of a frame which includes a source of error and a radiation exposure dose of the subject throughout the continuous radiological imaging is obtained based on the calculated E.I. of the entire radiation moving picture signal as described above, so that an accurate radiation exposure dose of the subject may be obtained. Further, this requires only a simple calculation and the processing load may be reduced.

The total radiation exposure dose obtained in the radiation exposure dose obtaining unit 142 in the manner described above is inputted to the radiation exposure dose management apparatus 150, and the radiation exposure dose management apparatus 150 registers the total radiation exposure dose with the ID information of the patient inputted in advance (S132). Then, the radiation exposure dose management apparatus 150 displays the registered total radiation exposure dose with the ID information of the patient as required or calculates a cumulative radiation exposure dose from the past for a particular patient and displays a warning message if the cumulative radiation exposure dose is greater than a predetermined specified value.

So far, a preferred embodiment of the second radiation exposure dose obtaining apparatus of the present invention has been described, but the second radiation exposure dose obtaining apparatus of the present invention is not limited to the aforementioned embodiment. For example, the signal evaluation value Rf of a radiation image signal of predetermined one frame required for the calculation by the formula (1) is not limited to the signal evaluation value of the radiation image signal of the first frame of the radiation moving picture signal described in the aforementioned embodiment. The signal evaluation value may be obtained by equipping the radiation image capturing apparatus 10 with a dose measurement switch (not shown), then when storing a radiation image signal of a frame immediately after the dose measurement switch is depressed during the continuous radiological imaging in the radiation image storage unit 13, appending information which indicates that this frame is the reference frame at the time of calculating the radiation exposure dose in the radiation image signal of the frame, and employing a signal evaluation value calculated based on the radiation image signal of the frame to which the information is appended as the signal evaluation value. Further, any other form may be employed as long as it is a signal evaluation value of a radiation image signal for one frame deemed to be free of error factor, such as a maximum signal evaluation value of those calculated for each radiation image signal, a signal evaluation value of one particular frame which does not include any error factor such as the contrast agent described above, an average value of signal evaluation values of radiation image signals of a plurality of frames that do not include any error factor, or the like.

Further, the radiation exposure dose obtaining apparatus is implemented as an independent apparatus in the embodiment described above, but it may be implemented in any other form, such as being incorporated in the other apparatus, such as the radiation image capturing apparatus, as a part thereof. More specifically, it may be provided in a console which includes the system control apparatus 60 or in the radiation image capturing apparatus 10. Further, in the case where the radiation image detector 12 is accommodated in a portable electronic cassette, and hardware of electronic circuits, such as LSI (Large Scale Integration), hardware of programmable electronic circuits, such as PLD (Programmable Logic Device) · FPGA (Field-Programmable Gate Array), and the like are accommodated in the electronic cassette, the identification of a contrast agent frame and acquisition of radiation exposure dose may be performed by the hardware. Such arrangement allows pursuit of more real time implementation.

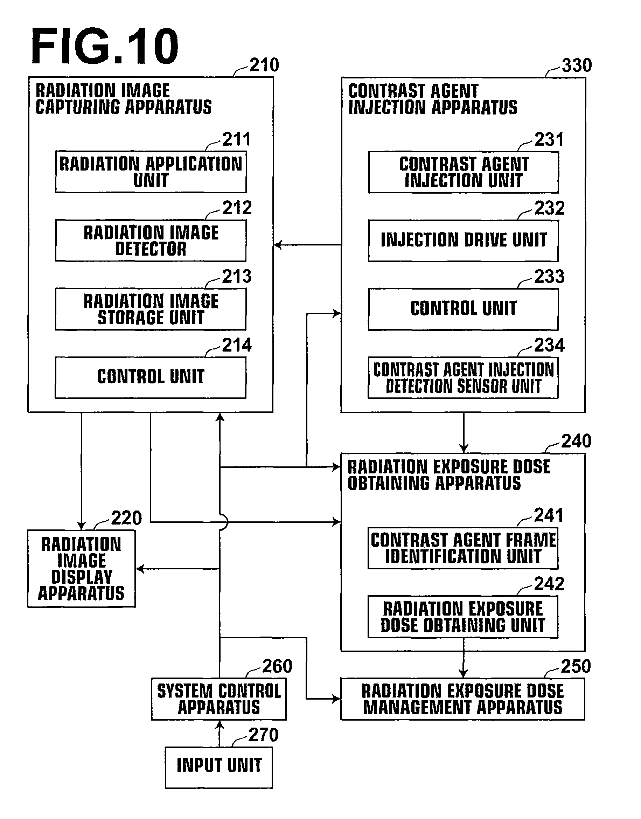

An embodiment of a radiation image capturing system that uses an embodiment of the third radiation exposure dose obtaining apparatus of the present invention will now be described with reference to the accompanying drawings. Figure 10 is a block diagram of the radiation image capturing system of the present embodiment, illustrating an overall schematic configuration thereof.

As illustrated in Figure 10, the radiation image capturing system of the present embodiment includes a radiation image capturing apparatus 210 which captures a fluoroscopic image (moving picture) of a patient, a radiation image display apparatus 220 which displays the fluoroscopic image captured by the radiation image capturing apparatus 210, a contrast agent injection apparatus 230 which injects a contrast agent into a blood vessel or a lymph vessel of the patient, a radiation exposure dose obtaining apparatus 240 which obtains an exposure dose of the patient based on an image signal of the fluoroscopic image captured by the radiation image capturing apparatus 210, a radiation exposure dose management apparatus 250 which stores and manages the exposure dose of each patient obtained by the radiation exposure dose obtaining apparatus 240, and a system control apparatus 260 which performs overall control of the radiation image capturing system.

As illustrated in Figure 10, the radiation image capturing apparatus 210 includes a radiation application unit 211 that has an X-ray tube, an aperture stop, and the like and applies radiation emitted from the x-ray tube and passed through the aperture stop to a patient, a radiation image detector 212 that detects radiation transmitted through the patient and outputs a radiation image signal representing a radiation image of the patient, a radiation image storage unit 213 that stores the radiation image signal outputted from the radiation image detector 212, and a control unit 214 that performs overall control of the radiation image capturing apparatus 210.

The radiation image detector 212 allows repeated use for recording and reading of radiation images, and a so-called direct type radiation image detector that records a radiation image by generating and accumulating a charge by directly receiving radiation or a so-called indirect type radiation image detector that records a radiation image by converting radiation first to visible light and then converting the visible light to a charge and accumulating the charge may be used. As for the radiation image signal reading method for reading out the radiation image recorded by accumulating charges in the manner described above, a so-called TFT (thin film transistor) reading method in which a radiation image signal is read by ON/OFF switching thin film transistors and a so-called optical reading method in which a radiation image signal is read out by applying reading light are preferably used, but others may also be used.

The radiation image capturing apparatus 210 is used to capture a fluoroscopic image (moving picture) of a subject to be injected with a contrast agent by the contrast agent injection apparatus 230, and the control unit 214 controls the radiation application unit 211 and radiation image detector 212 such that the fluoroscopic image is captured. More specifically, the control unit 214 controls the radiation application unit 211 to apply radiation at a predetermined frame rate and the radiation image detector 212 to perform recording and reading of a radiation image by the application of the radiation. Then, the radiation image signals of each frame outputted from the radiation image detector 212 are sequentially stored in the radiation image storage unit 213. Further, the control unit 214 of the present embodiment appends information indicating whether or not an image signal representing an image of the contrast agent is included in the radiation image signals of each frame when storing the radiation image signals of each frame in the radiation image storage unit 213, the operation of which will be described later in detail.

As for the structure of the radiation image capturing apparatus 210, a structure in which image capturing is performed with the patient being in the upright position or in the lateral position may be employed. Further, a structure that allows image capturing with the patient being both in upright position and lateral position may be employed.

The radiation image display apparatus 220 generates a display control signal by performing predetermined processing on the radiation image signals of each frame read out from the radiation image storage unit 213 of the radiation image capturing apparatus 210 and displays a fluoroscopic image of the patient on the monitor based on the display control signal.

The contrast agent injection apparatus 230 automatically injects a contrast agent into a patient based on an instruction input from the user or based on a preset injection start timing of the contrast agent. More specifically, the contrast agent injection apparatus 230 includes a contrast agent injection unit 231 formed of, for example, a syringe and injects a contrast agent into a blood vessel of the patient or the like, an injection drive unit 232 which drives the injection operation of the contrast agent injection unit 31, a control unit 233 that controls the operation of the injection drive unit 232, and a contrast agent injection detection sensor unit 234 that detects whether or not the contrast agent is being injected into a blood vessel or the like of the patient from the contrast agent injection apparatus 230.

The injection drive unit 232 moves, for example, the piston of the syringe of the contrast agent injection unit 231 and is formed of a power means, such as an air pressure, motor, or hydraulic pressure.

The contrast agent injection detection sensor unit 234 includes a sensor for detecting that the contrast agent is discharged from the contrast agent injection unit 231. Then, the contrast agent injection detection sensor unit 234 outputs a contrast agent detection signal to the control unit 214 of the radiation image capturing apparatus 210.

The radiation exposure dose obtaining apparatus 240 includes a contrast agent frame identification unit 241 that identifies a radiation image signal of a frame which includes an image signal of an image of a contrast agent as a contrast agent frame from the radiation image signal of each frame read out from the radiation image storage unit 213 of the radiation image capturing apparatus 210, and a radiation exposure dose obtaining unit 242 that obtains a radiation exposure dose of the patient based on a radiation moving picture signal which is the collection of the radiation image signals of each frame read out from the radiation image storage unit 213 of the radiation image capturing apparatus 210. Operations of the contrast agent frame identification unit 241 and the radiation exposure dose obtaining unit 242 will be described later in detail.

The radiation exposure dose management apparatus 250 stores the radiation exposure dose obtained by the radiation exposure dose obtaining apparatus 240 and manages the radiation exposure dose of each patient.

The system control apparatus 260 outputs control signals to the radiation image capturing apparatus 210, the radiation image display apparatus 220, the contrast agent injection apparatus 230, the radiation exposure dose obtaining apparatus 240, and the radiation exposure dose management apparatus 250 to control the operations thereof and at the same time performs input/output signal control between these apparatuses. The system control apparatus 260 is provided with an input unit 270 which receives an instruction input and other inputs, such as image capturing conditions, patient ID information and the like, from the user. The input information received by the input unit 270 is outputted to each apparatus by the system control apparatus 260 as required.

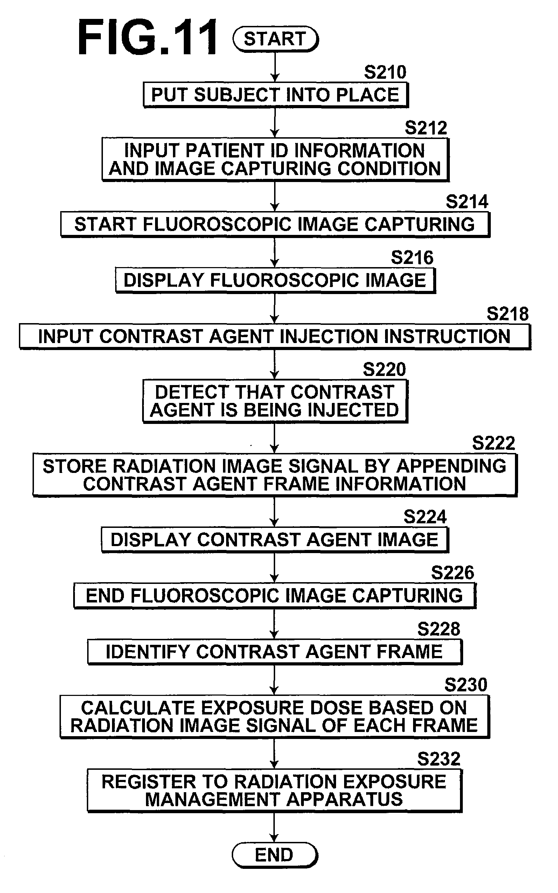

An operation of the radiation image capturing system of the present embodiment will now be described with reference to the flowchart of Figure 11.

First, a patient, the subject of the image capturing, is placed on an image capturing platform or the like provided in the radiation image capturing apparatus 210 and the patient is put into position (S210).

Then, ID information of the image capturing target patient and an image capturing condition are inputted by the user through the input unit 270 and the patient ID information is registered in the radiation exposure dose management apparatus 250 while the image capturing condition is set to the control unit 214 of the radiation image capturing apparatus 210 (S212). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user through the input unit 270 and in response to the input, a control signal is outputted from the system control apparatus 260 to the radiation image capturing apparatus 210 to capture a fluoroscopic image, and the radiation image capturing apparatus 210 starts fluoroscopic image capturing according to the inputted control signal (S214).

More specifically, the X-ray tube of the radiation application unit 211 is controlled based on the inputted imaging condition and a predetermined dose of radiation is applied toward the patient intermittently at a predetermined frame rate.

Then, radiation transmitted through the patient is applied to the radiation image detector 212 and subjected to a photoelectrical conversion in the radiation image detector 212 and accumulated therein as a charge signal.

Thereafter, each time the application of radiation for each frame is ended, the charge signal accumulated in the radiation image detector 212 is read out by the control unit 214, then converted to a digital signal by an A/D converter (not shown), and stored in the radiation image storage unit 213.

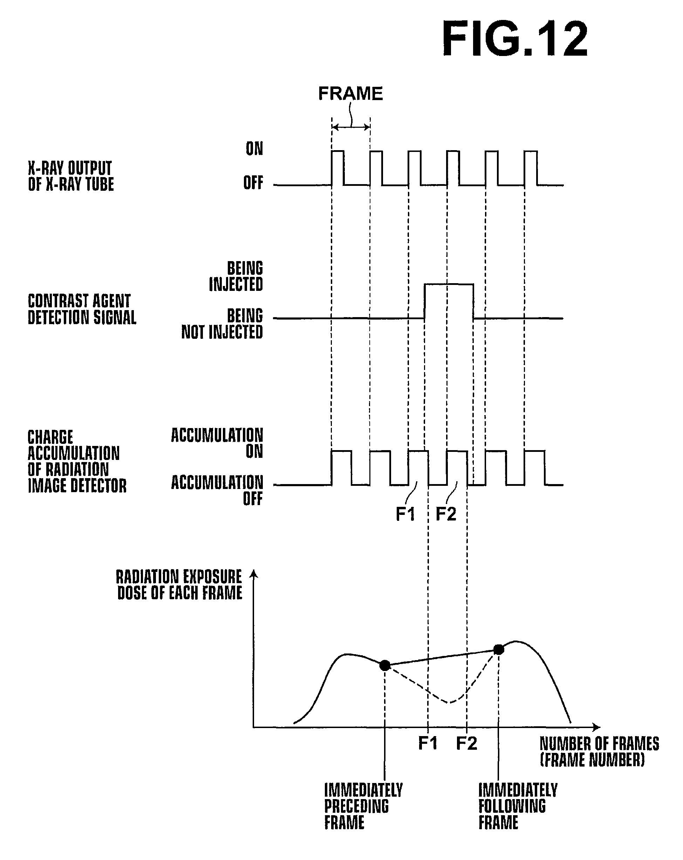

Figure 12 is a timing chart illustrating the application timing of radiation from the X-ray tube of the radiation application unit 211 and the charge accumulation timing of the radiation image detector 212. The period during which no charge accumulation is performed in the radiation image detector 212 (accumulation OFF period) is a period for reading out a charge signal from the radiation image detector 212.

The repeated performance of radiation application by the X-ray tube and the radiation image recording and reading in the radiation image detector 212 at a predetermined frame rate in the manner described above causes the radiation image signal of each frame to be sequentially stored in the radiation image storage unit 213.

Then, the radiation image signals of each frame stored in the radiation image storage apparatus 213 are sequentially read out and outputted to the radiation image display apparatus 220. The radiation image display apparatus 220 sequentially generates display control signals based on the inputted radiation image signals of each frame and sequentially outputs the display control signals to the monitor to display a fluoroscopic image of the patient as a moving picture (S216). It is assumed here that the radiation image signals with respect each frame stored in the radiation image storage unit 213 will remain un-erased in the radiation image storage unit 213 after being read out.

Here, if the user desires to observe a contrast agent image, such as a blood vessel or the like, while the fluoroscopic image capturing and display are performed in the manner described above, a contrast agent injection instruction is inputted through the input unit 270 (S218).

When the contrast agent injection instruction is inputted through the input unit 270, a control signal is outputted from the system control apparatus 260 to the contrast agent injection apparatus 230. The contrast agent injection apparatus 230 starts contrast agent injection based on the inputted control signal. More specifically, the contrast agent injection unit 231 is driven by the injection drive unit 232 and a predetermined amount of contrast agent is discharged from the contrast agent injection unit 231.

Here, the contrast agent injection detection sensor unit 234 provided in the contrast agent injection apparatus 230 detects that the contrast agent is discharged and a detection signal is outputted to the control unit 214 of the radiation image capturing apparatus 210 (S220). When the contrast agent detection signal is inputted, the control unit 214 of the radiation image capturing apparatus 210 appends to the radiation image signal of a frame captured while the contrast agent detection signal is inputted information indicating that it includes an image signal representing an image of the contrast agent (hereinafter, referred to as "contrast agent frame information") as header information, and stores the radiation image signal in the radiation image storage unit 213 with the header information (S222). More specifically, if the contrast agent detection signal is inputted to the radiation image capturing apparatus 210 in the timing illustrated in Figure 12, the contrast agent frame information is appended to the radiation image signals of frames F1 and F2 captured while the contrast agent is injected and stored in the radiation image storage unit 213.

Then, the radiation image signals captured while the contrast agent is injected are also read out from the radiation image storage unit 213 and sequentially outputted to the radiation image display apparatus 220. The radiation image display apparatus 220 sequentially generates display control signals based on the inputted radiation image signals, and displays radiation images including a contrast agent image on the monitor based on the display control signals (S224).

Then, after the discharge of the predetermined amount of contrast agent from the contrast agent injection apparatus 230 is completed and the contrast agent detection signal is not detected any more in the contrast agent injection detection sensor unit 234, radiation image signals captured are stored in the radiation image storage unit 213 without the contrast agent frame information being appended, and radiation image signals with respect each frame are read out from the radiation image storage unit 213 in the manner described above and a fluoroscopic image is displayed on the radiation image display apparatus 220.

Thereafter, when an instruction to end the fluoroscopic image capturing is inputted by the user through the input unit 270, the system control apparatus 260 outputs a control signal to the radiation image capturing apparatus 210 to end the fluoroscopic image capturing, and the radiation image capturing apparatus 210 ends the fluoroscopic image capturing in response to the inputted control signal (S226).

When the fluoroscopic image capturing described above is ended, the system control apparatus 260 reads out radiation image signals of all frames, i.e., the radiation moving picture signal, stored in the radiation image storage unit 213 of the radiation image capturing apparatus 210, and inputs the radiation image signals to the radiation exposure dose obtaining apparatus 240.

Then, first, the contrast agent frame identification unit 241 identifies a radiation image signal of a frame to which contrast agent frame information is appended from the radiation moving picture signal and identifies the frame as a contrast agent frame (S228).

Next, the radiation exposure dose obtaining unit 242 calculates a radiation exposure dose received by the patient during capturing of the fluoroscopic image based on the radiation image signals of each frame (S230).

More specifically, an E.I. (Exposure Index) is calculated as a signal evaluation value for each frame based on a radiation image signal of each frame other than a contrast agent frame.

The E.I. is calculated in the following manner. First, a predetermined calculation area is set in a radiation image of each frame. The calculation area may be, for example, the entire area of the radiation image, an area arbitrarily set by the user, an area defined based on the imaged region information, or an area within 10% of the image size from the center of the radiation image. Otherwise, an area except for the so-called direct exposure area which may be obtained based on, for example, a histogram of the radiation image or an area of 90% of the entire density width from the central density of the radiation image may be employed. Alternatively, a specific calculation area may be set by combining the aforementioned conditions.

Next, a representative value V of the calculation area set in the above is calculated. As for the representative value V, the density value itself of the radiation image or a statistical characteristic value obtained by modifying the density value itself with the average value of the entire density values, median value, mode value, or trimmed mean value may be employed. Then, based on the representative value V, the E.I. of each frame is calculated by the formula given below:

$$E.I. = C_0 \times g\ (V),$$

where:

g(V): reverse calibration function; and
$C_0$: 100·Gy (constant).

Here, g(V) is a function defined based on a radiation image obtained with the radiation quality of RQA5. The representative value V differs in magnitude due to difference in sensitivity arising from difference in scintillator used in the radiation image detector or difference in the setting method of the calculation area or the calculation method of the representative value V and g(V) is a function to normalize the differences. That is, if RQA5 radiation is received by any type of radiation image detector by the same radiation dose, the E.I. will become nearly the same value.

As described above, an E.I. is calculated first for each frame, then an E.I. of the radiation moving picture signal (collection of radiation image signals of each frame) is calculated, and a radiation exposure dose of the subject due to the continuous radiological imaging is obtained based on the calculated E.I. of the radiation moving picture signal. In the case where a contrast agent is injected and a contrast agent image is captured in the middle of the fluoroscopic image capturing as described above, the radiation applied to the patient is absorbed by the contrast agent before reaching the radiation image detector 212, as illustrated in the graph shown at the bottom of Figure 12. Therefore, if a radiation exposure dose is calculated based on a radiation image signal of a contrast agent frame, the calculated value becomes smaller than that actually received by the patient.

The graph at the bottom of Figure 12 illustrates the radiation exposure dose of each frame connected by a line and the horizontal axis of the graph indicates the frame number from the start of imaging. More specifically, if a radiation exposure dose is calculated based on the radiation image signals of frames F1, F2 illustrated in Figure 12, the calculated radiation exposure dose will become smaller than the actual value (estimated value close to the line connecting the frames immediately preceding and following the frames F1, F2 which includes a contrast agent image).

Consequently, in the present embodiment, an E.I. of the radiation moving picture signal (collection of radiation image signals of each frame) is calculated by the formula (11) and a radiation exposure dose of the subject is obtained based on the calculated E.I. of the radiation moving picture signal. Note that the same value can be obtained by the formula (13).

$$Rm = Rs \times N/(N-M) \quad ----------- \quad (11)$$

$$Rm = Ra \times N \quad ---------------- \quad (13),$$

where:

Rm: E.I. of the entire radiation moving picture signal (signal evaluation value of the entire radiation moving picture signal);
N: number of frames of the entire radiation moving picture signal;
M: number of contrast agent frames in the radiation moving picture signal;
Rs: sum of E. I. of the radiation image signals of each frame other than a contrast agent frame; and
Ra: average value of E. I. of the radiation image signals of each frame other than a contrast agent frame.

The formulae (11), (13) calculate an E.I, of the entire radiation moving picture signal without using an E.I, of a contrast agent frame and interpolating the E.I. for the contrast agent frame using E.I. of the frames other than the contrast agent frame.

Then, a radiation exposure dose of the subject due to the continuous radiological imaging is obtained using the E.I. of the entire radiation moving picture signal calculated in the manner described above. As for the method of obtaining the radiation exposure dose using the E.I., for example, a function which defines these relationships or a lookup table may be set in advance.

The total radiation exposure dose obtained in the radiation exposure dose obtaining unit 242 is inputted to the radiation exposure dose management apparatus 250, and the radiation exposure dose management apparatus 250 registers the total radiation exposure dose for each body region of the patient with the ID information of the patient inputted in advance (S232). Then, the radiation exposure dose management apparatus 250 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the present embodiment, a frame which includes an image signal representing an image of a contrast agent is identified as a contrast agent frame from the radiation image signal of each frame, then a signal evaluation value of the radiation moving picture is calculated by the formula (11) or (13) which does not use information of the contrast agent frame, and a radiation exposure dose of the subject throughout the continuous radiological imaging is obtained based on the calculated signal evaluation value of the radiation moving picture signal. This allows more accurate radiation exposure dose of a subject which takes into account the inclusion of an image of a contrast agent in the radiation image to be obtained.

So far, a preferred embodiment of the third radiation exposure dose obtaining apparatus of the present invention has been described, but the third radiation exposure dose obtaining apparatus of the present invention is not limited to the aforementioned embodiment. For example, a contrast agent frame may be identified by any method other than the aforementioned method that uses the contrast agent injection detection sensor described above, such as a method in which a contrast agent injection time obtaining unit is provided and injection start and end time of the contrast agent are obtained by the contrast agent injection time obtaining unit and a contrast agent frame is obtained based on the time information, a method which identifies a frame whose signal evaluation value is within a predetermined range and differs from that of a frame other than the contrast agent from as a contrast agent frame without using these sensors, or the like.

Further, the radiation exposure dose obtaining apparatus is implemented as an independent apparatus in the embodiment described above, but it may be implemented in any other form, such as being incorporated in the other apparatus, such as the radiation image capturing apparatus, as a part thereof. More specifically, it may be provided in a console which includes the system control apparatus 260 or in the radiation image capturing apparatus 210. Further, in the case where the radiation image detector 212 is accommodated in a portable electronic cassette, and hardware of electronic circuits, such as LSI (Large Scale Integration), hardware of programmable electronic circuits, such as PLD (Programmable Logic Device) · FPGA (Field-Programmable Gate Array), and the like are accommodated in the electronic cassette, the identification of a contrast agent frame and acquisition of radiation exposure dose may be performed by the hardware. Such arrangement allows pursuit of more real time implementation.

An embodiment of a radiation image capturing system that uses an embodiment of the fourth radiation exposure dose obtaining apparatus of the present invention will now be described with reference to the accompanying drawings.

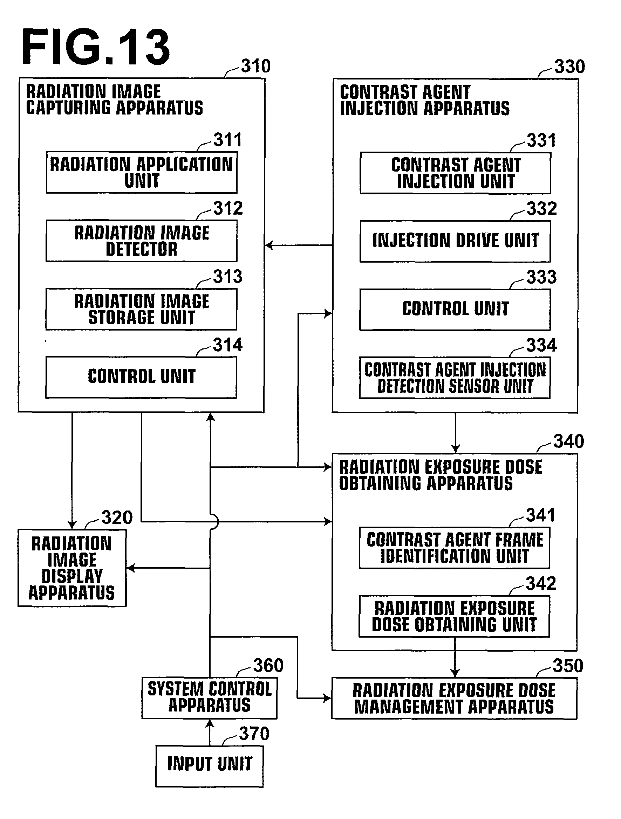

Figure 13 is a block diagram of the radiation image capturing system of the present embodiment, illustrating an overall schematic configuration thereof.

As illustrated in Figure 13, the radiation image capturing system of the present embodiment includes a radiation image capturing apparatus 310 which captures a fluoroscopic image (moving picture) of a patient, a radiation image display apparatus 320 which displays the fluoroscopic image captured by the radiation image capturing apparatus 210, a contrast agent injection apparatus 330 which injects a contrast agent into a blood vessel or a lymph vessel of the patient, a radiation exposure dose obtaining apparatus 340 which obtains an exposure dose of the patient based on an image signal of the fluoroscopic image captured by the radiation image capturing apparatus 310, a radiation exposure dose management apparatus 350 which stores and manages the exposure dose of each patient obtained by the radiation exposure dose obtaining apparatus 340, and a system control apparatus 360 which performs overall control of the radiation image capturing system.

As illustrated in Figure 10, the radiation image capturing apparatus 310 includes a radiation application unit 311 that has an X-ray tube, an aperture stop, and the like and applies radiation emitted from the x-ray tube and passed through the aperture stop to a patient, a radiation image detector 312 that detects radiation transmitted through the patient and outputs a radiation image signal representing a radiation image of the patient, a radiation image storage unit 313 that stores the radiation image signal outputted from the radiation image detector 312, and a control unit 314 that performs overall control of the radiation image capturing apparatus 310.

The radiation image detector 312 allows repeated use for recording and reading of radiation images, and a so-called direct type radiation image detector that records a radiation image by generating and accumulating a charge by directly receiving radiation or a so-called indirect type radiation image detector that records a radiation image by converting radiation first to visible light and then converting the visible light to a charge and accumulating the charge may be used. As for the radiation image signal reading method for reading out the radiation image recorded by accumulating charges in the manner described above, a so-called TFT (thin film transistor) reading method in which a radiation image signal is read by ON/OFF switching thin film transistors and a so-called optical reading method in which a radiation image signal is read out by applying reading light are preferably used, but others may also be used.

The radiation image capturing apparatus 310 is used to capture a fluoroscopic image (moving picture) of a subject to be injected with a contrast agent by the contrast agent injection apparatus 330, and the control unit 314 controls the radiation application unit 311 and radiation image detector 312 such that the fluoroscopic image is captured. More specifically, the control unit 314 controls the radiation application unit 311 to apply radiation at a predetermined frame rate and the radiation image detector 312 to perform recording and reading of a radiation image by the application of the radiation. Then, the radiation image signals of each frame outputted from the radiation image detector 312 are sequentially stored in the radiation image storage unit 313. Further, the control unit 314 of the present embodiment appends information indicating whether or not an image signal representing an image of the contrast agent is included in the radiation image signals of each frame when storing the radiation image signals of each frame in the radiation image storage unit 313, the operation of which will be described later in detail.

As for the structure of the radiation image capturing apparatus 310, a structure in which image capturing is performed with the patient being in the upright position or in the lateral position may be employed. Further, a structure that allows image capturing with the patient being both in upright position and lateral position may be employed.

The radiation image display apparatus 320 generates a display control signal by performing predetermined processing on the radiation image signals of each frame read out from the radiation image storage unit 313 of the radiation image capturing apparatus 310 and displays a fluoroscopic image of the patient on the monitor based on the display control signal.

The contrast agent injection apparatus 330 automatically injects a contrast agent into a patient based on an instruction input from the user or based on a preset injection start timing of the contrast agent. More specifically, the contrast agent injection apparatus 330 includes a contrast agent injection unit 331 formed of, for example, a syringe and injects a contrast agent into a blood vessel of the patient or the like, an injection drive unit 332 which drives the injection operation of the contrast agent injection unit 31, a control unit 333 that controls the operation of the injection drive unit 332, and a contrast agent injection detection sensor unit 334 that detects whether or not the contrast agent is being injected into a blood vessel or the like of the patient from the contrast agent injection apparatus 330.

The injection drive unit 332 moves, for example, the piston of the syringe of the contrast agent injection unit 331 and is formed of a power means, such as an air pressure, motor, or hydraulic pressure.

The contrast agent injection detection sensor unit 334 includes a sensor for detecting that the contrast agent is discharged from the contrast agent injection unit 331. Then, the contrast agent injection detection sensor unit 334 outputs a contrast agent detection signal to the control unit 314 of the radiation image capturing apparatus 310.

The radiation exposure dose obtaining apparatus 340 includes a contrast agent frame identification unit 341 that identifies a radiation image signal of a frame which includes an image signal of an image of a contrast agent as a contrast agent frame from the radiation image signal of each frame read out from the radiation image storage unit 313 of the radiation image capturing apparatus 310, and a radiation exposure dose obtaining unit 342 that obtains a radiation exposure dose of the patient based on a signal evaluation value of a radiation image signals of each frame read out from the radiation image storage unit 313 of the radiation image capturing apparatus 310. Operations of the contrast agent frame identification unit 341 and the radiation exposure dose obtaining unit 342 will be described later in detail.

The radiation exposure dose management apparatus 350 stores the radiation exposure dose obtained by the radiation exposure dose obtaining apparatus 340 and manages the radiation exposure dose for each patient.

The system control apparatus 360 outputs control signals to the radiation image capturing apparatus 310, the radiation image display apparatus 320, the contrast agent injection apparatus 330, the radiation exposure dose obtaining apparatus 340, and the radiation exposure dose management apparatus 350 to control the operations thereof and at the same time performs input/output signal control between these apparatuses. The system control apparatus 360 is provided with an input unit 370 which receives an instruction input and other inputs, such as image capturing conditions, patient ID information and the like, from the user. The input information received by the input unit 370 is outputted to each apparatus by the system control apparatus 360 as required.

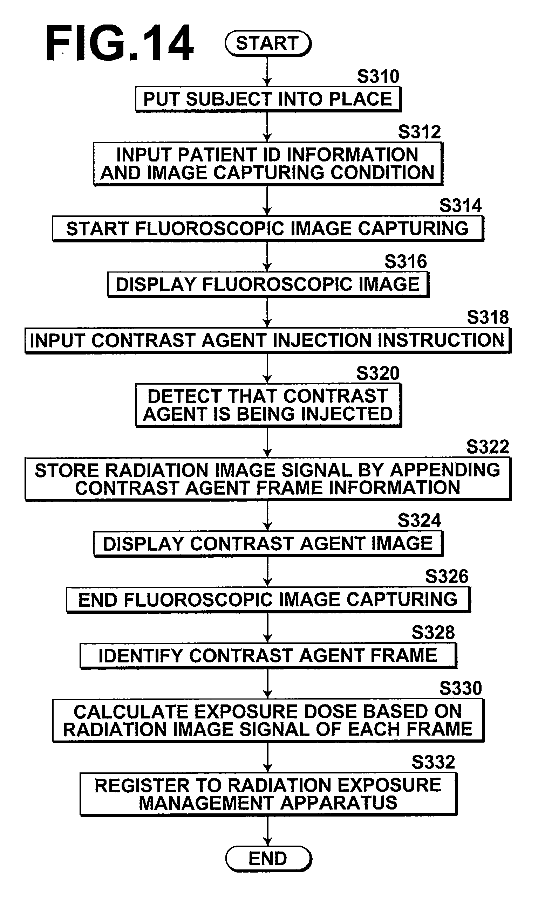

An operation of the radiation image capturing system of the present embodiment will now be described with reference to the flowchart of Figure 14.

First, a patient, the subject of the image capturing, is placed on an image capturing platform or the like provided in the radiation image capturing apparatus 310 and the patient is put into position (S310).

Then, ID information of the image capturing target patient and an image capturing condition are inputted by the user through the input unit 370 and the patient ID information is registered in the radiation exposure dose management apparatus 350 while the image capturing condition is set to the control unit 314 of the radiation image capturing apparatus 310 (S312). The image capturing condition may include a tube voltage, tube current, and application time in order to apply an appropriate dose of radiation to an image capturing region of the patient, as well as a frame rate for capturing a fluoroscopic image. As for the frame rate for capturing the fluoroscopic image, for example, a frame rate of 5fps to 60fps is set.

Next, an instruction to start fluoroscopic image capturing of the patient is inputted by the user through the input unit 370 and in response to the input, a control signal is outputted from the system control apparatus 360 to the radiation image capturing apparatus 310 to capture a fluoroscopic image, and the radiation image capturing apparatus 310 starts fluoroscopic image capturing according to the inputted control signal (S314).

More specifically, the X-ray tube of the radiation application unit 311 is controlled based on the inputted imaging condition and a predetermined dose of radiation is applied toward the patient intermittently at a predetermined frame rate.

Then, radiation transmitted through the patient is applied to the radiation image detector 312 and subjected to a photoelectrical conversion in the radiation image detector 312 and accumulated therein as a charge signal.

Thereafter, each time the application of radiation for each frame is ended, the charge signal accumulated in the radiation image detector 312 is read out by the control unit 314, then converted to a digital signal by an A/D converter (not shown), and stored in the radiation image storage unit 313.

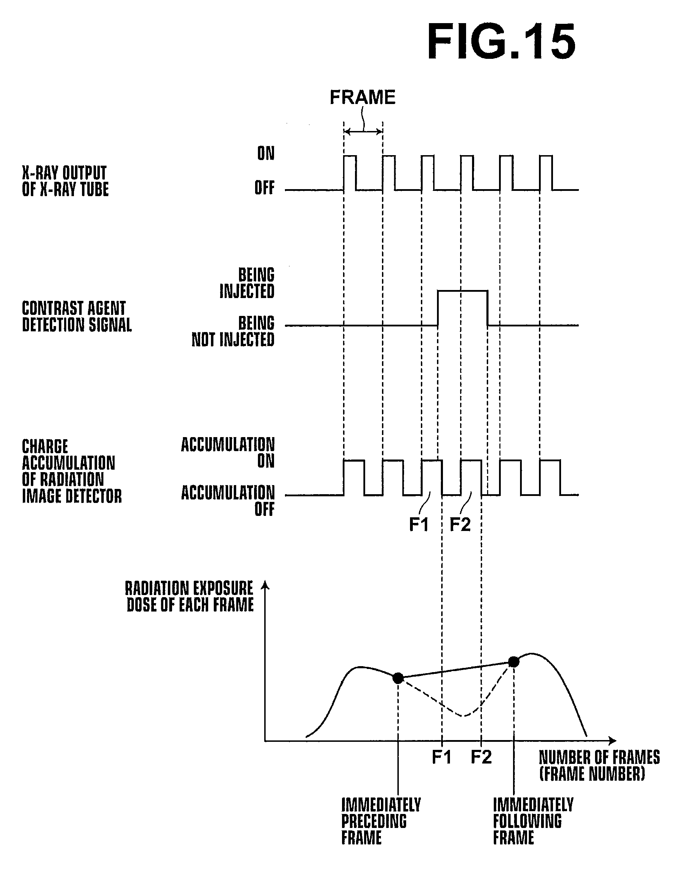

Figure 15 is a timing chart illustrating the application timing of radiation from the X-ray tube of the radiation application unit 311 and the charge accumulation timing of the radiation image detector 312. The period during which no charge accumulation is performed in the radiation image detector 312 (accumulation OFF period) is a period for reading out a charge signal from the radiation image detector 312.

The repeated performance of radiation application by the X-ray tube and the radiation image recording and reading in the radiation image detector 312 at a predetermined frame rate in the manner described above causes the radiation image signal of each frame to be sequentially stored in the radiation image storage unit 313.

Then, the radiation image signals of each frame stored in the radiation image storage apparatus 313 are sequentially read out and outputted to the radiation image display apparatus 320. The radiation image display apparatus 320 sequentially generates display control signals based on the inputted radiation image signals of each frame and sequentially outputs the display control signals to the monitor to display a fluoroscopic image of the patient as a moving picture (S316). It is assumed here that the radiation image signals with respect each frame stored in the radiation image storage unit 313 will remain un-erased in the radiation image storage unit 313 after being read out.

Here, if the user desires to observe a contrast agent image, such as a blood vessel or the like, while the fluoroscopic image capturing and display are performed in the manner described above, a contrast agent injection instruction is inputted through the input unit 370 (S318).

When the contrast agent injection instruction is inputted through the input unit 370, a control signal is outputted from the system control apparatus 360 to the contrast agent injection apparatus 330. The contrast agent injection apparatus 330 starts contrast agent injection based on the inputted control signal. More specifically, the contrast agent injection unit 331 is driven by the injection drive unit 332 and a predetermined amount of contrast agent is discharged from the contrast agent injection unit 331.

Here, the contrast agent injection detection sensor unit 334 provided in the contrast agent injection apparatus 330 detects that the contrast agent is discharged and a detection signal is outputted to the control unit 314 of the radiation image capturing apparatus 310 (S320). When the contrast agent detection signal is inputted, the control unit 314 of the radiation image capturing apparatus 310 appends to a radiation image signal of a frame captured while the contrast agent detection signal is inputted information indicating that it includes an image signal representing an image of the contrast agent (hereinafter, referred to as "contrast agent frame information") as header information, and stores the radiation image signal in the radiation image storage unit 313 with the header information (S322). More specifically, if the contrast agent detection signal is inputted to the radiation image capturing apparatus 310 in the timing illustrated in Figure 15, the contrast agent frame information is appended to the radiation image signals of frames F1 and F2 captured while the contrast agent is injected and stored in the radiation image storage unit 313.

Then, the radiation image signals captured while the contrast agent is injected are also read out from the radiation image storage unit 313 and sequentially outputted to the radiation image display apparatus 320. The radiation image display apparatus 320 sequentially generates display control signals based on the inputted radiation image signals, and displays radiation images including a contrast agent image on the monitor based on the display control signals (S324).

Then, after the discharge of the predetermined amount of contrast agent from the contrast agent injection apparatus 330 is completed and the contrast agent detection signal is not detected any more in the contrast agent injection detection sensor unit 334, radiation image signals captured are stored in the radiation image storage unit 313 without the contrast agent frame information being appended, and radiation image signals with respect each frame are read out from the radiation image storage unit 313 in the manner described above and a fluoroscopic image is displayed on the radiation image display apparatus 320.

Thereafter, when an instruction to end the fluoroscopic image capturing is inputted by the user through the input unit 370, the system control apparatus 360 outputs a control signal to the radiation image capturing apparatus 310 to end the fluoroscopic image capturing, and the radiation image capturing apparatus 310 ends the fluoroscopic image capturing in response to the inputted control signal (S326).

When the fluoroscopic image capturing described above is ended, the system control apparatus 360 reads out radiation image signals of all frames stored in the radiation image storage unit 313 of the radiation image capturing apparatus 310 and inputs the radiation image signals to the radiation exposure dose obtaining apparatus 340.

Then, first, the contrast agent frame identification unit 341 identifies a radiation image signal of a frame to which contrast agent frame information is appended from radiation image signal of all frames and identifies the frame as a contrast agent frame (S328).

Next, the radiation exposure dose obtaining unit 342 calculates a radiation exposure dose received by the patient during capturing of each frame of the fluoroscopic image based on the radiation image signals of each frame (S330). More specifically, an E.I. (Exposure Index) is calculated as a signal evaluation value based on the radiation image signals of each frame in the present embodiment and based on the E.I., the radiation exposure dose is obtained.

The E.I. is calculated in the following manner. First, a predetermined calculation area is set in a radiation image of each frame. The calculation area may be, for example, the entire area of the radiation image, an area arbitrarily set by the user, an area defined based on the imaged region information, or an area within 10% of the image size from the center of the radiation image. Otherwise, an area except for the so-called direct exposure area which may be obtained based on, for example, a histogram of the radiation image or an area of 90% of the entire density width from the central density of the radiation image may be employed. Alternatively, a specific calculation area may be set by combining the aforementioned conditions.

Next, a representative value V of the calculation area set in the above is calculated. As for the representative value V, the density value itself of the radiation image or a statistical characteristic value obtained by modifying the density value itself with the average value of the entire density values, median value, mode value, or trimmed mean value may be employed. Then, based on the representative value V, the E.I. of each frame is calculated by the formula given below:

(A)

$$\text{E.I.} = C_0 \times g\,(V),$$

(B)
where:

    $g(V)$: reverse calibration function; and

(C)
$C_0$: 100·Gy (constant).

Here, g(V) is a function defined based on a radiation image obtained with the radiation quality of RQA5. The representative value V differs in magnitude due to difference in sensitivity arising from difference in scintillator used in the radiation image detector or difference in the setting method of the calculation area or the calculation method of the representative value V and g(V) is a function to normalize the differences. That is, if RQA5 radiation is received by any type of radiation image detector by the same radiation dose, the E.I. will become nearly the same value.

A radiation exposure dose received by the patient during capturing of each frame is obtained using the E.I. calculated in the radiation exposure dose obtaining unit 342 based on the radiation image signals of each frame in the manner described above. As for the method of obtaining the radiation exposure dose using the E.I., for example, a function which defines these relationships or a lookup table may be set in advance.

The graph at the bottom of Figure 15 illustrates the radiation exposure dose obtained in the manner described above for each frame connected by a line and the horizontal axis of the graph indicates the frame number from the start of imaging.

Here, in the case where a contrast agent is injected and a contrast agent image is captured in the middle of the fluoroscopic image capturing as described above, the radiation applied to the patient is absorbed by the contrast agent before reaching the radiation image detector 312. Therefore, if a radiation exposure dose is calculated based on the radiation image signal of the contrast agent frame, the calculated value will become smaller than that actually received by the patient. More specifically, if a radiation exposure dose is calculated based on the radiation image signals of frames F1, F2 illustrated in Figure 15, the calculated radiation exposure dose will become smaller than the actual value (estimated value close to the line connecting the frames immediately preceding and following the frames F1, F2 which includes a contrast agent image).

Consequently, in the present embodiment, a value corrected by linear interpolation using a radiation exposure dose of a frame other than a contrast agent frame immediately preceding the contrast agent frame and a radiation exposure dose of a frame other than a contrast agent frame immediately following the contrast agent frame is used. More specifically, linear interpolation is performed using radiation exposure doses calculated based on radiation image signals of the frames captured immediately before and after the frames F1, F2 which includes a contrast agent image, as illustrated in the graph of Figure 15, and radiation exposure doses at the imaging timing of the contrast agent frames F1, F2 on the linear line are obtained, whereby radiation exposure doses of the patient during the capturing of the contrast agent frames F1, F2 are obtained..

Then, the radiation exposure dose obtaining unit 342 calculates a total radiation exposure dose of the subject throughout the continuous radiological imaging by adding radiation exposure doses of all frames.

The total radiation exposure dose obtained in the radiation exposure dose obtaining unit 342 in the manner described above is inputted to the radiation exposure dose management apparatus 350, and the radiation exposure dose management apparatus 350 registers the total radiation exposure dose with the ID information of the patient inputted in advance (S332). Then, the radiation exposure dose management apparatus 350 displays the registered total radiation exposure dose with the ID information of the patient as required or calculates a cumulative radiation exposure dose from the past for a particular patient and displays a warning message if the cumulative radiation exposure dose is greater than a predetermined specified value.

According to the radiation image capturing system of the present embodiment, a frame which includes an image signal representing an image of a contrast agent is identified as a contrast agent frame from the radiation image signal of each frame and, for a radiation exposure dose of a contrast agent image, a value corrected by linear interpolation using a radiation exposure dose of a frame other than a contrast agent frame immediately preceding the contrast agent frame and a radiation exposure dose of a frame other than a contrast agent frame immediately following the contrast agent frame is used. This allows more accurate radiation exposure dose of a subject which takes into account the inclusion of an image of a contrast agent in the radiation image to be obtained.

So far, a preferred embodiment of the fourth radiation exposure dose obtaining apparatus of the present invention has been described, but the fourth radiation exposure dose obtaining apparatus of the present invention is not limited to the aforementioned embodiment. For example, a contrast agent frame may be identified by any method other than the aforementioned method that uses the contrast agent injection detection sensor described above, such as a method in which a contrast agent injection time obtaining unit is provided and injection start and end time of the contrast agent are obtained by the contrast agent injection time obtaining unit and a contrast agent frame is obtained based on the time information, a method which identifies a frame whose signal evaluation value is within a predetermined range and differs from that of a frame other than the contrast agent from as a contrast agent frame without using these sensors, or the like.

Further, the radiation exposure dose obtaining apparatus is implemented as an independent apparatus in the embodiment described above, but it may be implemented in any other form, such as being incorporated in the other apparatus, such as the radiation image capturing apparatus, as a part thereof. More specifically, it may be provided in a console which includes the system control apparatus 360 or in the radiation image capturing apparatus 310. Further, in the case where the radiation image detector 312 is accommodated in a portable electronic cassette, and hardware of electronic circuits, such as LSI (Large Scale Integration), hardware of programmable electronic circuits, such as PLD (Programmable Logic Device) · FPGA (Field-Programmable Gate Array), and the like are accommodated in the electronic cassette, the identification of a contrast agent frame and acquisition of radiation exposure dose may be performed by the hardware. Such arrangement allows pursuit of more real time implementation.

Further, it should be appreciated that, other than the first to fourth embodiment described above, various modifications and alterations may be made without departing from the spirit of the present invention.

**Claims**

1. A radiation exposure dose obtaining apparatus, comprising:

   a radiation exposure dose obtaining unit that obtains, based on a radiation image signal of each frame detected by a radiation image detector through continuous radiological imaging of a subject, a radiation exposure dose of the subject due to the continuous radiological imaging; and
   an area setting unit that sets one or more areas corresponding to a region of the subject in an image represented by the radiation image signal,
   wherein the radiation exposure dose obtaining unit obtains the radiation exposure dose for each area.

2. The radiation exposure dose obtaining apparatus of claim 1, wherein the area setting unit segments the image represented by the radiation image signal into a plurality of predetermined segmented areas and associates each segmented area with a region of the subject.

3. The radiation exposure dose obtaining apparatus of claim 1, wherein the area setting unit identifies an area corresponding to a region of the subject in the image represented by the radiation image signal by image recognition processing and associates the identified area with the region of the subject.

4. The radiation exposure dose obtaining apparatus of any of claims 1 to 3, comprising a contrast agent frame identification unit that identifies a frame which includes an image signal representing an image of a contrast agent injected into the subject as a contrast agent frame from the radiation image signal of each frame, wherein
   the radiation exposure dose obtaining unit performs a correction on a radiation exposure dose for the contrast agent frame to bring the radiation exposure dose close to an actual value.

5. The radiation exposure dose obtaining apparatus of any of claims 1 to 4, wherein the radiation exposure dose obtaining unit obtains the radiation exposure dose based on an E.I. (Exposure Index) of the radiation image signal.

6. A radiation exposure dose obtaining method that obtains, based on a radiation image signal detected by a radiation image detector through continuous radiological imaging of a subject, a radiation exposure dose of the subject due to the continuous radiological imaging, the method comprising the steps of:

   setting one or more areas corresponding to a region of the subject in an image represented by the radiation image signal; and
   obtaining the radiation exposure dose for each area.

7. A program for causing a computer to perform the radiation exposure dose obtaining method of claim 6.

8. A radiation exposure dose obtaining apparatus, comprising a radiation exposure dose obtaining unit that obtains, based on a radiation moving picture signal, which is a collection of a radiation image signal of each frame detected by a radiation image detector through continuous radiological imaging of a subject, a radiation exposure dose of the subject due to the continuous radiological imaging,
   wherein the radiation exposure dose obtaining unit calculates a signal evaluation value that serves as an index for determining how much of radiation is detected over the entire radiation moving picture signal by a formula (1) and obtains the radiation exposure dose of the subject based on the signal evaluation value of the radiation moving picture signal:

$$Rm = Rf \times N \quad ----------- \quad (1),$$

where:

Rm: the signal evaluation value of the radiation moving picture signal;
N: the number of frames of the entire radiation moving picture signal; and
Rf: the signal evaluation value of the radiation image signal of a predetermined one frame in the radiation moving picture signal.

9. The radiation exposure dose obtaining apparatus of claim 8, wherein the signal evaluation value of the radiation image signal of a predetermined one frame is a maximum signal evaluation value among those calculated for each radiation image signal.

10. The radiation exposure dose obtaining apparatus of claim 8, wherein the signal evaluation value of the radiation image signal of a predetermined one frame is a signal evaluation value of a radiation image signal of the first frame of the radiation moving picture signal.

11. The radiation exposure dose obtaining apparatus of any of claims 8 to 10, wherein the signal evaluation value of the radiation image signal of a predetermined one frame is a value calculated based on an E.I. (Exposure Index).

12. A radiation exposure dose obtaining method that obtains, based on a radiation moving picture signal, which is a collection of a radiation image signal of each frame detected by a radiation image detector through continuous radiological imaging of a subject, a radiation exposure dose of the subject due to the continuous radiological imaging, the method comprising the steps of:

calculating a signal evaluation value that serves as an index for determining how much of radiation is detected over the entire radiation moving picture signal by a formula (1); and
obtaining the radiation exposure dose of the subject based on the signal evaluation value of the radiation moving picture signal:

$$Rm = Rf \times N \quad \text{------------} \quad (1),$$

where:

Rm: the signal evaluation value of the radiation moving picture signal;
N: the number of frames of the entire radiation moving picture signal; and
Rf: the signal evaluation value of the radiation image signal of a predetermined one frame in the radiation moving picture signal.

13. A program for causing a computer to perform the radiation exposure dose obtaining method of claim 12.

14. A radiation exposure dose obtaining apparatus, comprising:

a radiation exposure dose obtaining unit that obtains, based on a radiation image signal of each frame detected by a radiation image detector through continuous radiological imaging of a subject to be injected with a contrast agent, a radiation exposure dose of the subject due to the continuous radiological imaging; and
a contrast agent frame identification unit that identifies a frame which includes an image signal representing an image of the contrast agent as a contrast agent frame from the radiation image signal of each frame,
wherein the radiation exposure dose obtaining unit calculates a signal evaluation value of a radiation moving picture signal, which is a collection of the radiation image signal of each frame, by a formula (11) and obtains the radiation exposure dose of the subject due to the continuous radiological imaging based on the signal evaluation value of the radiation moving picture signal,

$$Rm = Rs \times N / (N-M) \quad \text{-----------} \quad (11)$$

where:

Rm: the signal evaluation value of the radiation moving picture signal;
N: the number of frames of the entire radiation moving picture signal;
M: the number of contrast agent frames in the radiation moving picture signal;
Rs: the sum of signal evaluation value of radiation image signal of each frame other than the contrast agent frame.

15. The radiation exposure dose obtaining apparatus of claim 14, wherein the signal evaluation value of the radiation image signal of each frame is a value calculated based on an E.I. (Exposure Index) .

16. A radiation exposure dose obtaining method that obtains, based on a radiation image signal of each frame detected by a radiation image detector through continuous radiological imaging of a subject to be injected with a contrast agent, a radiation exposure dose of the subject due to the continuous radiological imaging, the method comprising the steps of:

identifying a frame which includes an image signal representing an image of the contrast agent as a contrast agent frame from the radiation image signal of each frame;
calculating a signal evaluation value of a radiation moving picture signal, which is a collection of the radiation image signal of each frame, by a formula (11); and
obtaining the radiation exposure dose of the subject due to the continuous radiological imaging based on the signal evaluation value of the radiation moving picture signal:

$$Rm = Rs{\times}N/(N{-}M) \quad {-}{-}{-}{-}{-}{-}{-}{-}{-}{-}{-} \quad (11)$$

where:

Rm: the signal evaluation value of the radiation moving picture signal;
N: the number of frames of the entire radiation moving picture signal;
M: the number of contrast agent frames in the radiation moving picture signal;
Rs: the sum of signal evaluation value of radiation image signal of each frame other than the contrast agent frame.

17. A program for causing a computer to perform the radiation exposure dose obtaining method of claim 16.

18. A radiation exposure dose obtaining apparatus, comprising:

a radiation exposure dose obtaining unit that obtains, based on a radiation image signal of each frame detected by a radiation image detector through continuous radiological imaging of a subject to be injected with a contrast agent, a radiation exposure dose of the subject due to the continuous radiological imaging; and
a contrast agent frame identification unit that identifies a frame which includes an image signal representing an image of the contrast agent as a contrast agent frame from the radiation image signal of each frame,
wherein the radiation exposure dose obtaining unit obtains, for the radiation exposure dose of the contrast agent frame, a value corrected by linear interpolation using a radiation exposure value of a frame other than the contrast agent frame adjacent to the contrast agent frame on the front side and a radiation exposure value of a frame other than the contrast agent frame adjacent to the contrast agent frame on the back side.

19. The radiation exposure dose obtaining apparatus of claim 18, wherein the radiation exposure dose obtaining unit obtains, for the radiation exposure dose of the contrast agent frame, a value corrected by linear interpolation using a radiation exposure value of a frame other than the contrast agent frame immediately preceding the contrast agent frame and a radiation exposure value of a frame other than the contrast agent frame immediately following the contrast agent frame.

20. The radiation exposure dose obtaining apparatus of claim 18 or 19, wherein the radiation exposure dose obtaining unit obtains the radiation exposure dose based on an E.I. (Exposure Index) of the radiation image signal.

21. A radiation exposure dose obtaining method that obtains, based on a radiation image signal of each frame detected by a radiation image detector through continuous radiological imaging of a subject to be injected with a contrast agent, a radiation exposure dose of the subject due to the continuous radiological imaging, the method comprising the steps of:

identifying a frame which includes an image signal representing an image of the contrast agent as a contrast agent frame from the radiation image signal of each frame; and
obtaining, for the radiation exposure dose of the contrast agent frame, a value corrected by linear interpolation using a radiation exposure value of a frame other than the contrast agent frame adjacent to the contrast agent frame on the front side and a radiation exposure value of a frame other than the contrast agent frame adjacent to the contrast agent frame on the back side.

22. A program for causing a computer to perform the radiation exposure dose obtaining method of claim 21.

# FIG.1

**RADIATION IMAGE CAPTURING APPARATUS** /10

- RADIATION APPLICATION UNIT /11
- RADIATION IMAGE DETECTOR /12
- RADIATION IMAGE STORAGE UNIT /13
- CONTROL UNIT /14

**CONTRAST AGENT INJECTION APPARATUS** /30

- CONTRAST AGENT INJECTION UNIT /31
- INJECTION DRIVE UNIT /32
- CONTROL UNIT /33
- CONTRAST AGENT INJECTION DETECTION SENSOR UNIT /34

**RADIATION IMAGE DISPLAY APPARATUS** /20

**RADIATION EXPOSURE DOSE OBTAINING APPARATUS** /40

- AREA SETTING UNIT /41
- CONTRAST AGENT FRAME IDENTIFICATION UNIT /42
- RADIATION EXPOSURE DOSE OBTAINING UNIT /43

**SYSTEM CONTROL APPARATUS** /60

**INPUT UNIT** /70

**RADIATION EXPOSURE DOSE MANAGEMENT APPARATUS** /50

# FIG.2  (START)

**S10**
PUT SUBJECT INTO PLACE

**S12**
INPUT PATIENT ID INFORMATION
AND IMAGE CAPTURING CONDITION

**S14**
START FLUOROSCOPIC IMAGE CAPTURING

**S16**
DISPLAY FLUOROSCOPIC IMAGE

**S18**
INPUT CONTRAST AGENT INJECTION INSTRUCTION

**S20**
DETECT THAT CONTRAST
AGENT IS BEING INJECTED

**S22**
STORE RADIATION IMAGE SIGNAL BY APPENDING
CONTRAST AGENT FRAME INFORMATION

**S24**
DISPLAY CONTRAST AGENT IMAGE

**S26**
END FLUOROSCOPIC IMAGE CAPTURING

**S28**
SET AREA IN IMAGE

**S30**
IDENTIFY CONTRAST AGENT FRAME

**S32**
CALCULATE EXPOSURE DOSE FOR EACH AREA

**S34**
REGISTER TO RADIATION EXPOSURE
MANAGEMENT APPARATUS

(END)

# FIG.3

# FIG.4

RADIATION EXPOSURE DOSE
AREA 1

TIME

RADIATION EXPOSURE DOSE
AREA 4

TIME

RADIATION EXPOSURE DOSE
AREA 5

TIME

RADIATION EXPOSURE DOSE
AREA 6

TIME

# FIG.5

Ac

Al

EP 2 716 223 A1

TID 10001 PROJECTION X-RAY RADIATION DOSE  --+--- **DoseSR BASIC INFORMATION**

TID (10002) Observer Context

TID (1003) Person observer identifying attributes
or TID (1004) Device observer identifying attributes  ×1~n  ---+--- **INFORMATION OF IMAGE CAPTURING APPARATUS AND PRACTITIONER**

TID (10002) Accumulated X-Ray Dose  ×1~2 ---+--- **ACCUMULATED DOSE DATA**

TID (10004) Accumulated Projection X-Ray Dose

ACCUMULATED DOSE DATA
IN FIRST ACCUMULATION

PATIENT BODY REGION DATA
IN FIRST ACCUMULATION

:

ACCUMULATED DOSE DATA
IN $n^{th}$ ACCUMULATION

PATIENT BODY REGION DATA
IN SECOND ACCUMULATION

TID (10003) Irradiation Event X-Ray Data  ---+--- **APPLIED DOSE DATA: FOR NUMBER OF APPLICATIONS**

TID (1020) Person Participant  (option)  ---+--- **INFORMATION OF REQUESTED DOCTOR**

# FIG.6

# FIG.7

**RADIATION IMAGE CAPTURING APPARATUS** ⌐110

    **RADIATION APPLICATION UNIT** ⌐111

    **RADIATION IMAGE DETECTOR** ⌐112

    **RADIATION IMAGE STORAGE UNIT** ⌐113

    **CONTROL UNIT** ⌐114

**CONTRAST AGENT INJECTION APPARATUS** ⌐130

    **CONTRAST AGENT INJECTION UNIT** ⌐131

    **INJECTION DRIVE UNIT** ⌐132

    **CONTROL UNIT** ⌐133

    **CONTRAST AGENT INJECTION DETECTION SENSOR UNIT** ⌐134

**RADIATION IMAGE DISPLAY APPARATUS** ⌐120

**RADIATION EXPOSURE DOSE OBTAINING APPARATUS** ⌐140

    **RADIATION EXPOSURE DOSE OBTAINING UNIT** ⌐142

**RADIATION EXPOSURE DOSE MANAGEMENT APPARATUS** ⌐150

**SYSTEM CONTROL APPARATUS** ⌐160

**INPUT UNIT** ⌐170

# FIG.8 (START)

↓ S110
PUT SUBJECT INTO PLACE

↓ S112
INPUT PATIENT ID INFORMATION AND IMAGE CAPTURING CONDITION

↓ S114
START FLUOROSCOPIC IMAGE CAPTURING

↓ S116
DISPLAY FLUOROSCOPIC IMAGE

↓ S118
INPUT CONTRAST AGENT INJECTION INSTRUCTION

↓ S120
DETECT THAT CONTRAST AGENT IS BEING INJECTED

↓ S122
STORE RADIATION IMAGE SIGNAL BY APPENDING CONTRAST AGENT FRAME INFORMATION

↓ S124
DISPLAY CONTRAST AGENT IMAGE

↓ S126
END FLUOROSCOPIC IMAGE CAPTURING

↓ S128
IDENTIFY CONTRAST AGENT FRAME

↓ S130
CALCULATE EXPOSURE DOSE BASED ON RADIATION IMAGE SIGNAL OF EACH FRAME

↓ S132
REGISTER TO RADIATION EXPOSURE MANAGEMENT APPARATUS

↓
(END)

# FIG.9

FRAME

X-RAY OUTPUT
OF X-RAY TUBE

ON

OFF

CONTRAST AGENT
DETECTION SIGNAL

BEING
INJECTED

BEING
NOT INJECTED

CHARGE
ACCUMULATION
OF RADIATION
IMAGE DETECTOR

ACCUMULATION
ON

ACCUMULATION
OFF

F1   F2

RADIATION EXPOSURE
DOSE OF EACH FRAME

ESTIMATED VALUE
ACTUAL VALUE

F1   F2     NUMBER OF FRAMES
(FRAME NUMBER)

IMMEDIATELY
PRECEDING
FRAME

IMMEDIATELY
FOLLOWING
FRAME

# FIG.10

RADIATION IMAGE
CAPTURING APPARATUS /210

RADIATION APPLICATION
UNIT /211

RADIATION IMAGE
DETECTOR /212

RADIATION IMAGE
STORAGE UNIT /213

CONTROL UNIT /214

CONTRAST AGENT
INJECTION APPARATUS /330

CONTRAST AGENT
INJECTION UNIT /231

INJECTION DRIVE UNIT /232

CONTROL UNIT /233

CONTRAST AGENT INJECTION
DETECTION SENSOR UNIT /234

RADIATION
IMAGE
DISPLAY
APPARATUS /220

RADIATION EXPOSURE DOSE
OBTAINING APPARATUS /240

CONTRAST AGENT FRAME
IDENTIFICATION UNIT /241

RADIATION EXPOSURE
DOSE OBTAINING UNIT /242

SYSTEM CONTROL
APPARATUS /260

INPUT UNIT /270

RADIATION EXPOSURE DOSE
MANAGEMENT APPARATUS /250

# FIG.11

( START )

↓ S210
PUT SUBJECT INTO PLACE

↓ S212
INPUT PATIENT ID INFORMATION
AND IMAGE CAPTURING CONDITION

↓ S214
START FLUOROSCOPIC IMAGE CAPTURING

↓ S216
DISPLAY FLUOROSCOPIC IMAGE

↓ S218
INPUT CONTRAST AGENT INJECTION INSTRUCTION

↓ S220
DETECT THAT CONTRAST
AGENT IS BEING INJECTED

↓ S222
STORE RADIATION IMAGE SIGNAL BY APPENDING
CONTRAST AGENT FRAME INFORMATION

↓ S224
DISPLAY CONTRAST AGENT IMAGE

↓ S226
END FLUOROSCOPIC IMAGE CAPTURING

↓ S228
IDENTIFY CONTRAST AGENT FRAME

↓ S230
CALCULATE EXPOSURE DOSE BASED ON
RADIATION IMAGE SIGNAL OF EACH FRAME

↓ S232
REGISTER TO RADIATION EXPOSURE
MANAGEMENT APPARATUS

↓
( END )

# FIG.12

FRAME

**X-RAY OUTPUT
OF X-RAY TUBE**

ON

OFF

**CONTRAST AGENT
DETECTION SIGNAL**

BEING
INJECTED

BEING
NOT INJECTED

**CHARGE
ACCUMULATION
OF RADIATION
IMAGE DETECTOR**

ACCUMULATION
ON

ACCUMULATION
OFF

F1   F2

RADIATION EXPOSURE
DOSE OF EACH FRAME

F1   F2

NUMBER OF FRAMES
(FRAME NUMBER)

IMMEDIATELY
PRECEDING
FRAME

IMMEDIATELY
FOLLOWING
FRAME

# FIG.13

```
┌─ 310 ─────────────────────────┐        ┌─ 330 ─────────────────────────┐
│ RADIATION IMAGE               │        │ CONTRAST AGENT                │
│ CAPTURING APPARATUS           │        │ INJECTION APPARATUS           │
│                               │        │                               │
│   ┌─ 311 ──────────────────┐  │        │   ┌─ 331 ──────────────────┐  │
│   │ RADIATION APPLICATION  │  │        │   │ CONTRAST AGENT         │  │
│   │ UNIT                   │  │        │   │ INJECTION UNIT         │  │
│   └────────────────────────┘  │        │   └────────────────────────┘  │
│   ┌─ 312 ──────────────────┐  │        │   ┌─ 332 ──────────────────┐  │
│   │ RADIATION IMAGE        │  │        │   │ INJECTION DRIVE UNIT   │  │
│   │ DETECTOR               │  │        │   └────────────────────────┘  │
│   └────────────────────────┘  │        │   ┌─ 333 ──────────────────┐  │
│   ┌─ 313 ──────────────────┐  │        │   │ CONTROL UNIT           │  │
│   │ RADIATION IMAGE        │  │        │   └────────────────────────┘  │
│   │ STORAGE UNIT           │  │        │   ┌─ 334 ──────────────────┐  │
│   └────────────────────────┘  │        │   │ CONTRAST AGENT INJECTION│ │
│   ┌─ 314 ──────────────────┐  │        │   │ DETECTION SENSOR UNIT  │  │
│   │ CONTROL UNIT           │  │        │   └────────────────────────┘  │
│   └────────────────────────┘  │        └───────────────────────────────┘
└───────────────────────────────┘
```

RADIATION EXPOSURE DOSE OBTAINING APPARATUS — 340

CONTRAST AGENT FRAME IDENTIFICATION UNIT — 341

RADIATION EXPOSURE DOSE OBTAINING UNIT — 342

RADIATION IMAGE DISPLAY APPARATUS — 320

SYSTEM CONTROL APPARATUS — 360

INPUT UNIT — 370

RADIATION EXPOSURE DOSE MANAGEMENT APPARATUS — 350

# FIG.14

(START)

S310
PUT SUBJECT INTO PLACE

S312
INPUT PATIENT ID INFORMATION
AND IMAGE CAPTURING CONDITION

S314
START FLUOROSCOPIC IMAGE CAPTURING

S316
DISPLAY FLUOROSCOPIC IMAGE

S318
INPUT CONTRAST AGENT INJECTION INSTRUCTION

S320
DETECT THAT CONTRAST
AGENT IS BEING INJECTED

S322
STORE RADIATION IMAGE SIGNAL BY APPENDING
CONTRAST AGENT FRAME INFORMATION

S324
DISPLAY CONTRAST AGENT IMAGE

S326
END FLUOROSCOPIC IMAGE CAPTURING

S328
IDENTIFY CONTRAST AGENT FRAME

S330
CALCULATE EXPOSURE DOSE BASED ON
RADIATION IMAGE SIGNAL OF EACH FRAME

S332
REGISTER TO RADIATION EXPOSURE
MANAGEMENT APPARATUS

(END)

# FIG.15

FRAME

X-RAY OUTPUT
OF X-RAY TUBE

ON

OFF

CONTRAST AGENT
DETECTION SIGNAL

BEING
INJECTED

BEING
NOT INJECTED

CHARGE
ACCUMULATION
OF RADIATION
IMAGE DETECTOR

ACCUMULATION
ON

ACCUMULATION
OFF

F1    F2

RADIATION EXPOSURE
DOSE OF EACH FRAME

F1    F2    NUMBER OF FRAMES
(FRAME NUMBER)

IMMEDIATELY
PRECEDING
FRAME

IMMEDIATELY
FOLLOWING
FRAME

## INTERNATIONAL SEARCH REPORT

International application No.

PCT/JP2012/003559

A. CLASSIFICATION OF SUBJECT MATTER
*A61B6/00*(2006.01)i, *A61B6/10*(2006.01)i

According to International Patent Classification (IPC) or to both national classification and IPC

B. FIELDS SEARCHED

Minimum documentation searched (classification system followed by classification symbols)
A61B6/00, A61B6/10

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2007-215918 A (Shimadzu Corp.),<br>30 August 2007 (30.08.2007),<br>entire text; all drawings<br>(Family: none) | 1,6,7<br>2,5<br>4 |
| Y | JP 2008-132147 A (Toshiba Corp.),<br>12 June 2008 (12.06.2008),<br>entire text; all drawings<br>(Family: none) | 2 |
| Y | Eiji ARIGA, "(2)Kando Shihyo ni yoru Sochikan no Hibaku Senryo Hyoka ni Tsuite (2. Ippan Satsuei deno Gashitsu to Hibaku Senryo no Saitekika)", Keisoku Bunkakaishi, 18(2), 2010. 10, 14 to 16 | 5 |

☐ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>06 September, 2012 (06.09.12) | Date of mailing of the international search report<br>18 September, 2012 (18.09.12) |
|---|---|
| Name and mailing address of the ISA/<br>Japanese Patent Office | Authorized officer |
| Facsimile No. | Telephone No. |

Form PCT/ISA/210 (second sheet) (July 2009)

## INTERNATIONAL SEARCH REPORT

International application No.

PCT/JP2012/003559

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:
```
(Invention 1) the inventions of claims 1, 2 and 4-7
(Invention 2) the invention of claim 3
(Invention 3) the inventions of claims 8-13
(Invention 4) the inventions of claims 14-17
(Invention 5) the inventions of claims 18-22
```

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
   ```
   the inventions of claims 1, 2 and 4-7
   ```

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4387644 B